# EUROPEAN PATENT APPLICATION

(11) **EP 4 652 930 A1**
(43) Date of publication of application: **26.11.2025**
(21) Application number: 24877257.6
(22) Date of filing: 10.10.2024
(51) Int. Cl.: A61B 5/1455, A61B 10/00

(54) **BLOOD SUBSTANCE CONCENTRATION MEASUREMENT DEVICE, BLOOD SUBSTANCE CONCENTRATION MEASUREMENT METHOD, AND PROGRAM**

(30) Priority: 11.10.2023 JP 2023176173
(71) Applicant: Light Touch Technology Incorporated, Osaka-shi, Osaka, 540-0029 (JP)
(72) Inventor: YAMAKAWA, Koichi, Osaka-shi Osaka 540-0029 (JP); OGAWA, Kanade, Osaka-shi Osaka 540-0029 (JP); YAMAKAWA, Yoko, Osaka-shi Osaka 540-0029 (JP)
(74) Representative: Grünecker Patent- und Rechtsanwälte PartG mbB
(86) International application number: PCT/JP2024/036366
(87) International publication number: WO 2025/079663

(57) **Abstract**

A measuring unit 2, 3, 83, 84 that measures information relating to a target part of a subject person, a light emission unit 2 that emits a laser beam to a specific region in a subject portion Mp0 in the target part, a photodetector 3 that receives signal light reflected from the subject person and detects an intensity of the signal light, an optical member 4 that condenses the laser beam to the specific region in the subject portion Mp0 in the target part and forms, on the photodetector 3, a focused image of the signal light from a region to which the laser beam is condensed, an optical-member movable unit 5 that changes a state of the optical member 4, and a controller 9 that, based on the intensity of the signal light, obtains a concentration of a substance in blood in the region to which the laser beam is condensed are provided. The measuring unit 2, 3 measures the information relating to the target part, and the optical-member movable unit 5 changes the state of the optical members based on the information relating to the target part.

## Description

### [Technical Field]

The present disclosure relates to a device and a method for measuring, by a noninvasive measurement method, the concentration of substances that are included in blood flowing through blood vessels of a living body.

### [Background Art]

To prevent and treat lifestyle-related diseases, it is significant that the states of substances in blood, such as the blood sugar level and blood lipid level, be checked on a daily basis. In particular, for patients with diabetes, which is one of such lifestyle-related diseases, it is required that the concentration of glucose in blood be measured and the blood sugar level be managed on a daily basis in order to prevent complications, and an invasive method in which blood is sampled from a patient to chemically analyze the blood has been conventionally performed.

In contrast, in recent years, simple noninvasive methods have been proposed in which the state of blood in the body is optically analyzed without sampling blood (for example, Patent Literatures 1 and 2).

### [Citation List]

### [Patent Literature]

[Patent Literature 1] WO 2016/117520
[Patent Literature 2] JP 2009-168670A

### [Summary of Invention]

### [Technical Problem]

However, in the conventional noninvasive substance-in-blood concentration measurement device disclosed in each of Patent Literatures 1 and 2, measurement is performed using a finger as the measurement-target part in a state in which the palm side of the finger is placed in contact with a light-emission window provided on the outer surface of the measurement device. Thus, there is a problem that, because the subject person needs to be in contact with a part of the measurement device, it is difficult to measure concentrations of substances in blood in various scenes in daily life and from subject persons who are moving actively.

The present disclosure has been made in view of the above-described problem, and an object thereof is to provide a substance-in-blood concentration measurement device, a substance-in-blood concentration measurement method, and a program that make it possible to measure concentrations of substances in blood more simply and stably by using, as a measurement-target part, parts of the body of a subject person where the skin is exposed, which include not only the fingers but also other parts.

### [Solution to Problem]

In order to achieve the above-described object, a substance-in-blood concentration measurement device according to one aspect of the present disclosure is a substance-in-blood concentration measurement device that measures a concentration of a substance in blood included in the blood in a subject portion in a target part of a subject person, the substance-in-blood concentration measurement device being characterized by including: a measuring unit that measures information relating to the target part of the subject person; a light emission unit that emits a laser beam to a specific region in the subject portion in the target part; a photodetector that receives signal light reflected from the subject person based on the laser beam and detects an intensity of the signal light; an optical member that condenses the laser beam to the specific region in the subject portion in the target part, and forms, on the photodetector, a focused image of the signal light from a region to which the laser beam is condensed in the subject portion; an optical-member movable unit that changes a state of the optical member; and a controller that, based on the intensity of the signal light, obtains a concentration of the substance in blood in the region to which the laser beam is condensed, wherein the measuring unit measures the information relating to the target part of the subject person, and the optical-member movable unit changes the state of the optical member based on the information relating to the target part.

### [Advantageous Effects of Invention]

According to the substance-in-blood concentration measurement device, the substance-in-blood concentration measurement method, and the program according to aspects of the present disclosure, a substance-in-blood concentration measurement device, a substance-in-blood concentration measurement method, and a program can be provided that make it possible to measure concentrations of substances in blood more simply and stably by using, as a measurement-target part, parts of the body of a subject person where the skin is exposed, which include not only the fingers but also other parts.

### [Brief Description of Drawings]

FIG. 1 is a schematic diagram illustrating a state of a substance-in-blood concentration measurement device 1 according to Embodiment 1 during measurement.
FIG. 2 is a diagram illustrating a structure of the substance-in-blood concentration measurement device 1.
FIG. 3 is a flowchart illustrating a substance-in-blood measurement operation by the substance-in-blood concentration measurement device 1.
FIG. 4 is a diagram illustrating a structure of a substance-in-blood concentration measurement device 1A according to a modification, in which a light emission unit 2 has been modified into a different structure.
FIG. 5 is a diagram illustrating a structure of a substance-in-blood concentration measurement device 1B according to a modification, in which the light emission unit 2 has been modified into a different structure.
FIG. 6 is a diagram illustrating a structure of a substance-in-blood concentration measurement device 1C according to a modification, in which the light emission unit 2 has been modified into a different structure.
FIG. 7 is a diagram illustrating a different embodiment of a structure from an optical member 4 to a target part Ob in the substance-in-blood concentration measurement devices 1, 1A, 1B, and 1C.
FIG. 8A is a schematic diagram illustrating a state of a substance-in-blood concentration measurement device 1D according to Embodiment 2 during measurement, and FIG. 8B is a perspective view illustrating the substance-in-blood concentration measurement device 1D.
FIG. 9 is a horizontal cross-sectional view illustrating a structure of the substance-in-blood concentration measurement device 1D according to Embodiment 2.
FIG. 10 is a flowchart illustrating a substance-in-blood measurement operation by the substance-in-blood concentration measurement device 1D.
FIGS. 11A to 11C are explanatory diagrams for explaining an adjustment operation of the substance-in-blood concentration measurement device 1D.
FIG. 12 is a horizontal cross-sectional view for explaining the adjustment operation of the substance-in-blood concentration measurement device 1D.
FIG. 13 is a schematic diagram illustrating a structure of a substance-in-blood concentration measurement device 1E according to Embodiment 3.
FIG. 14 is a schematic diagram illustrating a structure of a substance-in-blood concentration measurement device 1F according to a modification.
FIG. 15 is a schematic diagram illustrating a structure of a substance-in-blood concentration measurement device 1G according to a modification.
FIG. 16A is a schematic diagram illustrating a structure of an example of the substance-in-blood concentration measurement device 1G, FIG. 16B is a diagram illustrating a relationship between positions of the target part Ob of a subject person measured using the example of the substance-in-blood concentration measurement device 1G and the intensity of signal light detected using the example of the substance-in-blood concentration measurement device 1G, and FIG. 16C is a diagram illustrating a temporal change in the blood sugar level of the subject person.
FIG. 17 is a schematic diagram illustrating a structure of a substance-in-blood concentration measurement device 1H according to a modification.
FIGS. 18A and 18B are diagrams for explaining operations by the substance-in-blood concentration measurement device 1H according to a modification.
FIGS. 19A and 19B are schematic diagrams illustrating structures of a light emission unit 2H and a photodetector 3H in the substance-in-blood concentration measurement device 1H according to a modification.
FIG. 20 is a schematic diagram illustrating a structure of a substance-in-blood concentration measurement device 1I according to a modification.
FIGS. 21A and 21B are schematic diagrams illustrating structures of a light emission unit 2I and a photodetector 3I in the substance-in-blood concentration measurement device 1I according to a modification.
FIGS. 22A and 22B are diagrams for explaining operations by the substance-in-blood concentration measurement device 1I according to a modification.
FIGS. 23A and 23B are diagrams for explaining the operations by the substance-in-blood concentration measurement device 1I according to a modification.
FIGS. 24A and 24B are schematic diagrams illustrating a structure of a substance-in-blood concentration measurement device 1J according to a modification.
FIG. 25 is a flowchart illustrating a substance-in-blood measurement operation by the substance-in-blood concentration measurement devices 1E, 1F, and 1J.
FIG. 26 is a flowchart illustrating a substance-in-blood measurement operation by the substance-in-blood concentration measurement devices 1G and 1H.
FIG. 27 is a flowchart illustrating a substance-in-blood measurement operation by the substance-in-blood concentration measurement device 1I.
FIGS. 28A to 28C are diagrams illustrating combination conditions of an incident angle of a laser beam L1 relative to the surface of the skin of the target part Ob and a light-receiving angle (installation angle) of a photodetector 3 relative to the surface of the skin of the target part Ob in an evaluation test.
FIG. 29 is a diagram illustrating results, for conditions 1 and 2, of a substance-in-blood concentration measurement test in which an example of the substance-in-blood concentration measurement device 1 and a comparative example based on Flash Glucose Monitoring (FGM) were used.
FIG. 30 is a diagram illustrating results, for conditions 3 and 4, of the substance-in-blood concentration measurement test, in which the example of the substance-in-blood concentration measurement device 1 and the FGM-based comparative example were used.
FIG. 31 is a diagram illustrating results, for conditions 5 and 6, of the substance-in-blood concentration measurement test, in which the example of the substance-in-blood concentration measurement device 1 and the FGM-based comparative example were used.

### [Description of Embodiments]

### <<Overview of Embodiments of Invention>>

A substance-in-blood concentration measurement device according to at least one embodiment of the present disclosure is a substance-in-blood concentration measurement device that measures a concentration of a substance in blood included in the blood in a subject portion in a target part of a subject person, the substance-in-blood concentration measurement device being characterized by including: a measuring unit that measures information relating to the target part of the subject person; a light emission unit that emits a laser beam to a specific region in the subject portion in the target part; a photodetector that receives signal light reflected from the subject person based on the laser beam and detects an intensity of the signal light; an optical member that condenses the laser beam to the specific region in the subject portion in the target part, and forms, on the photodetector, a focused image of the signal light from a region to which the laser beam is condensed in the subject portion; an optical-member movable unit that changes a state of the optical member; and a controller that, based on the intensity of the signal light, obtains a concentration of the substance in blood in the region to which the laser beam is condensed.

Furthermore, according to another aspect, in any of the above-described aspects, the optical-member movable unit may change the state of the optical member based on the information relating to the target part.

Furthermore, according to another aspect, in any of the above-described aspects, the measuring unit may include a distance-related-information measuring unit that measures, as the information relating to the target part, information that changes based on a distance to the target part from a specific position on optical paths of the laser beam and the signal light, the optical member may be present on an optical path shared by the laser beam and the signal light, and, based on the information measured by the distance-related-information measuring unit, the optical-member movable unit may transfer the optical member along the shared optical path to a position where the region to which the laser beam is condensed is capable of matching the specific region in the subject portion in the target part, and where a focused image of the signal light from the region to which the laser beam is condensed can be formed on the photodetector, whereafter the light emission unit may emit the laser beam, the photodetector may receive the signal light based on the laser beam and detect the intensity of the signal light, and the controller may measure the concentration of the substance in blood based on the intensity of the signal light.

According to such structures, the concentration of the subject in blood can be measured by an operator directing the distal-end portion of the substance-in-blood concentration measurement device toward the direction of the target part of the subject person and emitting the laser beam to the target part of the subject person. Thus, the concentration of the substance in blood can be measured more simply and stably by taking advantage of the characteristics of optical measurement and using, as the measurement-target part, parts of the body of a subject person where the skin is exposed, which include not only the fingers but also other parts such as the forehead portion of the subject person, for example.

Furthermore, according to another aspect, in any of the above-described aspects, the measuring unit may include a distance measuring unit that measures, as the information relating to the target part, a distance to the target part from a specific position on optical paths of the laser beam and the signal light, the optical member may include: a first optical member that condenses the laser beam to the specific region in the subject portion in the target part; and a second optical member that forms, on the photodetector, a focused image of the signal light from the region to which the laser beam is condensed in the subject portion, the optical-member movable unit may include: a first position changing unit that controls a position of the first optical member on the optical path of the laser beam; and a second position changing unit that controls a position of the second optical member on the optical path of the signal light, and, based on the distance measured by the distance measuring unit, the first position changing unit may transfer the first optical member to a position where the region to which the laser beam is condensed is capable of matching the specific region in the subject portion in the target part, and, based on the distance measured by the distance measuring unit, the second position changing unit may transfer the second optical member to a position where a focused image of the signal light from the region to which the laser beam is condensed can be formed on the photodetector, whereafter the light emission unit may emit the laser beam, the photodetector may receive the signal light based on the laser beam and detect the intensity of the signal light, and the controller may measure the concentration of the substance in blood based on the intensity of the signal light.

Furthermore, according to another aspect, in any of the above-described aspects, the measuring unit may include a position measuring unit that measures, as the information relating to the target part, a position of the target part relative to a specific position on optical paths of the laser beam and the signal light, the optical member may include: a first optical member that is positioned on the optical path of the laser beam and that condenses the laser beam to the specific region in the subject portion in the target part; and a second optical member that is positioned on the optical path of the signal light and that forms, on the photodetector, a focused image of the signal light from the region to which the laser beam is condensed in the subject portion in the target part, the optical-member movable unit may include: a first angle changing unit that changes an emission angle of the first optical member; and a second angle changing unit that changes a light-receiving angle of the second optical member, and, based on the position measured by the position measuring unit, the first angle changing unit may change the emission angle of the first optical member to a direction in which the region to which the laser beam is condensed is capable of matching the specific region in the subject portion in the target part, and, based on the position measured by the position measuring unit, the second angle changing unit may change the light-receiving angle of the second optical member to a direction in which a focused image of the signal light from the region to which the laser beam is condensed can be formed on the photodetector, whereafter the light emission unit may emit the laser beam, the photodetector may receive the signal light based on the laser beam and detect the intensity of the signal light, and the controller may measure the concentration of the substance in blood based on the intensity of the signal light.

According to such structures, the concentration of the substance in blood can be measured in a state in which the subject person is facing the front of the substance-in-blood concentration measurement device and an image of the target part of the subject person captured by a camera is being displayed on a display unit of a main-body portion. Thus, the concentration of the substance in blood can be measured more simply and stably by taking advantage of the characteristics of optical measurement and using, as the measurement-target part, not only the fingers but also the face, etc., of the subject person.

Furthermore, according to another aspect, in any of the above-described aspects, the measuring unit may include a detecting unit that detects, as the information relating to the target part, an area of presence of the target part of the subject person in a space, the optical-member movable unit may change a position and an angle of the optical member with respect to each of a plurality of positions in the space adaptively so that the region to which the laser beam is condensed is capable of matching the specific region in the subject portion and a focused image of the signal light from the region to which the laser beam is condensed can be formed on the photodetector, and, based on the area of presence of the target part detected by the detecting unit, the optical-member movable unit may select a measurement-target position from among the plurality of positions and change the position and the angle of the optical member so as to be adapted to the measurement-target position, whereafter the light emission unit may emit the laser beam, the photodetector may receive the signal light based on the laser beam and detect the intensity of the signal light, and the controller may measure the concentration of the substance in blood based on the intensity of the signal light.

According to such a structure, the concentration of the substance in blood can be measured while identifying, with higher accuracy, the target part of a subject person when the subject person is passing through a predetermined path, such as a passage inside a building or an athletic track, or when the subject person is passing through a predetermined region, such as an entrance to a building.

Furthermore, according to another aspect, in any of the above-described aspects, the measuring unit may include a detecting unit that detects, as the information relating to the target part, an area of presence of the target part of the subject person in a space, the optical-member movable unit may change a position and an angle of the optical member with respect to each of a plurality of positions in the space adaptively so that the region to which the laser beam is condensed is capable of matching the specific region in the subject portion in the target part and a focused image of the signal light from the region to which the laser beam is condensed can be formed on the photodetector, the detecting unit may detect the area of presence of the target part of the subject person, the optical-member movable unit may change the position and the angle of the optical member so as to be adapted to each of the plurality of positions, to each of the plurality of positions, the light emission unit may emit the laser beam, from each of the plurality of positions, the photodetector may receive the signal light based on the laser beam and detect the intensity of the signal light, and the controller may measure the concentration of the substance in blood based on the intensity of the obtained signal light, and further select a measurement-target position from among the plurality of positions based on the area of presence of the target part and select the concentration of the substance in blood corresponding to the measurement-target position.

According to such a structure, a distribution of the concentration of the substance in blood can be measured by setting all areas of the body of the subject person as the target part.

Furthermore, in another aspect, a substance-in-blood concentration measurement device according to at least one embodiment of the present disclosure is a substance-in-blood concentration measurement device that measures a concentration of a substance in blood included in the blood in a subject portion in a target part of a subject person, the substance-in-blood concentration measurement device including: a detecting unit that detects whether or not the target part of the subject person is present within a specific area in a space; a light emission unit that emits a laser beam to a specific region in the subject portion in the target part; a photodetector that receives signal light from the subject person based on the laser beam and detects an intensity of the signal light; an optical member that condenses the laser beam to the specific region in the subject portion in the target part located in the specific area, and forms, on the photodetector, a focused image of the signal light from a region to which the laser beam is condensed in the subject portion; and a controller that, based on the intensity of the signal light, obtains a concentration of the substance in blood in the region to which the laser beam is condensed, wherein, when the detecting unit detects that the target part is present within the specific area in the space, the light emission unit emits the laser beam, the photodetector receives the signal light based on the laser beam and detects the intensity of the signal light, and the controller measures the concentration of the substance in blood based on the intensity of the signal light.

According to such a structure, the device can be simplified by eliminating an angle adjustment function from the light emission unit and the photodetector, and the concentration of the substance in blood can be measured using a device that is lower in cost in a case such as that in which it is known that the target part of subject persons will pass through a predetermined target region.

Furthermore, according to another aspect, in any of the above-described aspects, positions of the light emission unit and the optical member relative to the subject portion may be set so that a blood-vessel region located inward of the epidermis in the subject portion and the region to which the laser beam is condensed by the optical member overlap, and positions of the photodetector and the optical member relative to the subject portion may be set so that an image of the signal light from the blood-vessel region overlapping the region to which the laser beam is condensed is transferred by the optical member to form a focused image on a light receiving surface of the photodetector.

Furthermore, according to another aspect, in any of the above-described aspects, within a section from the light emission unit to the surface of the subject portion in an optical path from the light emission unit to the subject portion, the laser beam may propagate through a space except within a section in which the laser beam passes through the optical member, and, within a section from the surface of the subject portion to a light receiving surface of the photodetector in an optical path from the subject portion to the photodetector, the signal light may propagate through a space except within a section in which the signal light passes through the optical member.

Furthermore, in another aspect, a substance-in-blood concentration measurement method according to at least one embodiment of the present disclosure is a substance-in-blood concentration measurement method for measuring a concentration of a substance in blood included in the blood in a subject portion in a target part of a subject person, the substance-in-blood concentration measurement method including: using a measuring unit and measuring information relating to the target part of the subject person; based on the information relating to the target part, using an optical-member movable unit and changing a state of an optical member; using a light emission unit and emitting a laser beam to a specific region in the subject portion in the target part; using the optical member and condensing the laser beam to the specific region in the subject portion in the target part, and forming, on a photodetector, a focused image of signal light from a region to which the laser beam is condensed in the subject portion; using the photodetector and receiving the signal light from the subject person based on the laser beam and detecting an intensity of the signal light; and, based on the intensity of the signal light, obtaining a concentration of the substance in blood in the region to which the laser beam is condensed.

According to such a structure, a substance-in-blood concentration measurement method can be realized that makes it possible to measure the concentration of the substance in blood more simply and stably by using, as the measurement-target part, parts of the body of the subject person where the skin is exposed, which include not only the fingers but also other parts.

Furthermore, in another aspect, a program relating to substance-in-blood concentration measurement processing according to at least one embodiment of the present disclosure is a program that causes a computer to execute substance-in-blood concentration measurement processing of measuring a concentration of a substance in blood included in the blood in a subject portion in a target part of a subject person, wherein the substance-in-blood concentration measurement processing includes: using a measuring unit and measuring information relating to the target part of the subject person; based on the information relating to the target part, using an optical-member movable unit and changing a state of an optical member; using a light emission unit and emitting a laser beam to a specific region in the subject portion in the target part; using the optical member and condensing the laser beam to the specific region in the subject portion in the target part, and forming, on a photodetector, a focused image of signal light from a region to which the laser beam is condensed in the subject portion; using the photodetector and receiving the signal light from the subject person based on the laser beam and detecting an intensity of the signal light; and, based on the intensity of the signal light, obtaining a concentration of the substance in blood in the region to which the laser beam is condensed.

According to such a structure, a program can be realized that makes it possible to measure the concentration of the substance in blood more simply and stably by using, as the measurement-target part, parts of the body of the subject person where the skin is exposed, which include not only the fingers but also other parts.

### <<Embodiment 1>>

A substance-in-blood concentration measurement device 1 according to the present embodiment will be described with reference to the drawings. Here, in this description, the positive and negative directions in the height direction may be respectively referred to as the "upward" and "downward" directions, and a surface facing the positive direction in the height direction and a surface facing the negative direction in the height direction may be respectively referred to as a "front" surface and a "rear" surface. Furthermore, members in the drawings are not necessarily drawn to scale. Furthermore, "perpendicular" and "parallel" may indicate angles with differences from the angles 90° and 0°, respectively, as long as functions are not substantially impaired thereby. Furthermore, in this description, numerical ranges indicated using the symbol "-" include the values at both ends of the numerical ranges. Further, only preferred examples of materials, numerical values, etc., are indicated in the present embodiment, and there is no limitation thereto.

### <Overall Structure>

The substance-in-blood concentration measurement device 1 (also referred to hereinafter as "device 1") according to Embodiment 1 is a device that noninvasively measures the concentration of a substance in the blood of a living body in a blood-vessel region by emitting a laser beam having a specific wavelength from a light source to the blood-vessel region of the living body and detecting the intensity of signal light from the blood-vessel region. As the laser beam, light of a specific wavelength capable of being absorbed by the substance to be measured is used. When the concentration of the substance in blood is high, signal light having lower light intensity than the laser beam in some wavelengths is emitted from the blood-vessel region due to absorption by the substance. Thus, the device 1 measures the concentration of the substance in blood by measuring the intensity of the signal light using a photodetector.

Conventionally, in most substance-in-blood concentration measurement devices, measurement is performed using the surface of a finger (living body) as a subject portion in a state in which the palm side of the finger is in contact with a measurement base. In contrast to this, the device 1 according to Embodiment 1 is characterized in that the device 1 makes it possible to measure the concentration of the substance in blood more simply using, as a measurement-target part, another part of the body of a subject person in place of a finger.

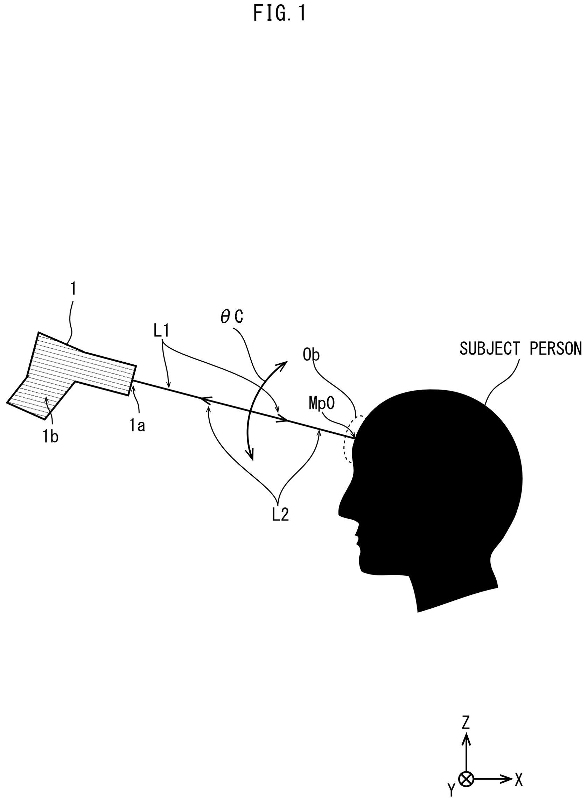

FIG. 1 is a schematic diagram illustrating a state of the device 1 during measurement.

When the forehead portion of the skin of the subject person is the target part Ob, for example, measurement using the substance-in-blood concentration measurement device 1 is performed by an operator emitting a laser beam L1 to the surface of the forehead of the subject person in a state in which a distal-end portion 1a of the device 1 is directed toward the direction of the forehead of the subject person. In doing so, in the state in which the distal-end portion 1a of the device 1 is directed toward the direction of the forehead of the subject person, the operator of the device 1 performs the measurement while holding a main-body portion 1b of the device 1 so that the angle formed by the emission direction of the laser beam L1 and the surface of the forehead of the subject person is within a predetermined range θc.

Thus, the laser beam L1 is emitted to a subject portion Mp0 present in the skin in the target part Ob from a built-in light source, and signal light L2 reflected from the subject portion Mp0 is received by a built-in photodetector, whereby the concentration of the substance in blood is measured by the device 1.

According to such a structure, the concentration of the substance in blood in the subject person can be measured more simply. Note that, with the device 1, measurement can also be performed using, as the target part Ob, other portions of the body of the subject person where the skin is exposed, besides the forehead.

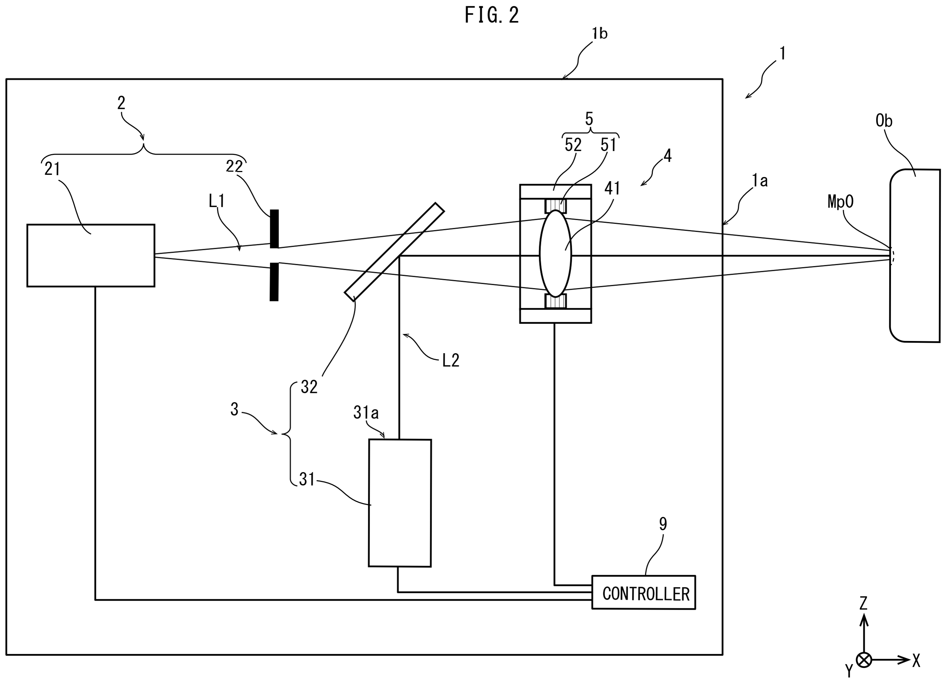

FIG. 2 is a schematic diagram illustrating a structure of the device 1.

As illustrated in FIG. 2, the device 1 includes a light emission unit 2, a photodetector 3, an optical member 4, an optical-member movable unit 5, and a controller 9, which are installed inside the main-body portion 1b of the device 1. Specifically, the light emission unit 2 is disposed in a position in which the laser beam L1 can be emitted from the distal-end portion 1a of the device 1, the optical member 4 is disposed on the optical path of the laser beam L1, the photodetector 3 is disposed on the optical path of the signal light L2 having passed through the optical member 4, and the optical-member movable unit 5 is mechanically connected to the optical member 4. Furthermore, the controller 9 is electrically connected to the light emission unit 2, the photodetector 3, and the optical-member movable unit 5.

In the following, structures of the elements of the device 1 will be described.

### <Structures of Elements>

### (Light Emission Unit 2)

The light emission unit 2 is a light source that emits the laser beam L1 having the specific wavelength to the living body toward the subject portion Mp0 in the target part Ob of the subject person. The light emission unit 2 includes a laser light source 21 that is capable of emitting a laser beam for target measurement (also referred to hereinafter as "target-measurement laser beam") that oscillates at a wavelength that is absorbed by the substance in blood that is the measurement-target substance (also referred to hereinafter as "target substance in blood").

Furthermore, in the device 1, the light emission unit 2 is capable of modulating laser-beam wavelength to selectively emit the laser beam for target measurement and a laser beam for measurement that oscillates at a wavelength that is absorbed by a different substance in blood. Thus, the type of substance in blood that can be detected is changed.

The laser light source 21 includes a light-emitting element that oscillates pulsed pump light having a shorter wavelength than mid-infrared light, and an optical parametric oscillator (OPO) that converts the wavelength into a longer wavelength and amplifies the pump light to emit the resultant light as a laser beam. For example, a Q-switched Nd:YAG laser (oscillation wavelength: 1.064 µm) or a Q-switched Yb:YAG laser (oscillation wavelength: 1.030 µm) may be used as the light-emitting element. A structure disclosed in a known document, e.g., the structure disclosed in JP 2010-281891, may be used for the OPO. Thus, the laser beam L1, which is higher in intensity compared to conventional light sources, can be obtained.

Glucose may be adopted, for example, as the substance in blood that is the measurement-target substance. In such a case, mid-infrared light, e.g., light having a wavelength selected from the range of 2.5-12 µm, inclusive, may be emitted as the laser beam. That is, as the wavelength oscillated by optical parametric oscillation, a predetermined wavelength selected from mid-infrared light may be adopted.

Thus, the glucose concentration in the blood of the subject can be measured as the blood sugar level. In this case, it is necessary to measure the glucose concentration in blood vessels in the skin, and mid-infrared light, which does not readily penetrate deeply into the body, is emitted directly to blood vessels (capillary vessels) in the skin. By identifying the position of blood vessels in the skin and emitting mid-infrared light thereto, an effect can be obtained that observation of only the blood-vessel portion is possible, in which case the observation is less susceptible to the influence of other body components present deeper in the body.

On the other hand, as the different substance in blood measured for adjustment before the target substance in blood is measured, a substance in blood satisfying the following is selected. That is, the substance has a higher laser-beam absorption rate than the measurement-target substance and thus has high measurement sensitivity, and the concentration of the substance in blood is very stable, resulting in less fluctuation in measurement results. The accuracy of the measurement of the measurement target can be improved by performing the target measurement after performing the measurement for adjustment with respect to a substance in blood having this structure. Hemoglobin can be selected as one example of such substance in blood.

By detecting hemoglobin in blood vessels, the positions of capillary vessels in the living body can be detected, and the most-suitable position of the measurement-target portion in the subject portion Mp0 in the target part Ob of the subject person for performing the measurement of the measurement-target substance can be specified. Here, the laser beam to be emitted to measure hemoglobin is light having a wavelength selected from mid-infrared light, and the wavelength is a predetermined wavelength selected from the range of 5.0-12 µm, inclusive. In order to change the wavelength of light emitted by the light emission unit 2, the configuration of the oscillation wavelength of the OPO in the light emission unit 2 is changed.

The light emission unit 2 may include a diaphragm 22 on the optical path of the laser beam L1. The diaphragm 22 is formed from a light-blocking plate-shaped member, and an opening (aperture) is opened in the center portion thereof. The center of the opening is aligned with the optical axis of the laser beam L1. Furthermore, the beam diameter of the laser beam L1 may be restricted to approximately one third by the opening, for example.

By the diaphragm 22 being provided to the light emission unit 2, the fluctuation in the emission position of the laser beam L1 emitted to the target part Ob of the subject person can be reduced. By using the diaphragm 22 in the optical path of the laser beam L1 as an image source, the target-measurement laser beam L1 can be used as guide light used in an adjustment process. By using light having the same wavelength as the target-measurement laser beam as the guide light in the adjustment process, it becomes unnecessary to correct a minute positioning error that would otherwise occur due to the difference in wavelength from the target-measurement laser beam.

The light emission unit 2 is electrically connected to the later-described controller 9, and outputs the laser beam L1 based on a control signal from the controller 9.

### (Photodetector 3)

The photodetector 3 is a light-receiving unit that receives the signal light L2, which is emitted from a laser-beam condensing region in the target part Ob of the subject person based on the emitted laser beam L1, and detects the intensity of the signal light L2. The photodetector 3 is disposed on the optical path of the signal light L2 having passed through the optical member 4, and is constituted from an infrared sensor 31 and a beam splitter 32.

The beam splitter 32 is a non-polarizing or polarizing beam splitter (branching optical element) that splits light at a fixed intensity ratio, and is disposed on the optical paths of the laser beam L1 and the signal light L2. The beam splitter 32 transmits the laser beam L1 emitted from the light emission unit 2 and guides the laser beam L1 to the subject portion Mp0 in the target part Ob, and also reflects the signal light L2 of reflection from the subject portion Mp0 and guides the signal light L2 to the infrared sensor 31.

The infrared sensor 31 is a near-infrared or mid-infrared sensor. A near-infrared sensor used as the infrared sensor 31 may have a sensitive wavelength range of 0.78-2.5 µm, inclusive, for example, and a mid-infrared sensor used as the infrared sensor 31 may have a sensitive wavelength range of 2.5-20 µm, inclusive, for example. The photodetector 3 outputs an electric signal corresponding to the intensity of the received signal light L2. For example, as the photodetector 3, a single-element infrared sensor that outputs the intensity of the signal light L2 as a one-dimensional voltage value, or a two-dimensional infrared sensor in which a plurality of elements are arranged in a matrix may be used. In the photodetector 3, the easily usable non-cooling method, electronic cooling method, or the like can be used.

In the device 1, the photodetector 3 can receive signal light having sufficiently high intensity relative to background light as a result of the intensity of the emitted laser beam L1 being increased by the light emission unit 2 and the signal light L2 from the laser-beam condensing region being imaged as a focused image on a light receiving surface 31a of the photodetector 3 by the later-described optical member 4; thus, a high signal-to-noise (S/N) ratio is realized and measurement can be performed with high accuracy.

The photodetector 3 is electrically connected to the later-described controller 9, and, based on a control signal from the controller 9, outputs the intensity of signal light received by the element to the controller 9 as a one-dimensional voltage value.

### (Optical Member 4)

The optical member 4 is disposed on the optical paths of the laser beam L1 and the signal light L2 in a state in which the optical member 4 is held by an optical-member holder 51. The optical member 4 is constituted from a condenser lens 41, and, as illustrated in FIG. 2, has the function of condensing emitted light to a specific region in the subject portion Mp0 in the optical path of the laser beam L1 from the light emission unit 2 to the target part Ob of the subject person.

The laser beam L1 having passed through the optical member 4 enters the target part Ob of the subject person and passes through the epithelial interstitial tissue of the living body to be scattered or diffusely reflected. The signal light L2 so produced is emitted toward the photodetector 3.

The optical member 4 also has the function of forming, on the photodetector 3, a focused image of the signal light L2 emitted as a result of diffuse reflection in the optical path of the signal light L2 from the subject portion Mp0 in the target part Ob of the subject person to the photodetector 3.

Here, in the present example, within a section of the forward optical path from the light emission unit 2 to the surface of the target part Ob of the subject person, the laser beam L1 propagates through a space filled with a gas, etc., for example, except within a section in which the laser beam L1 passes through the optical member 4. Similarly, within a section of the return optical path from the surface of the target part Ob of the subject person to the light receiving surface 31a of the photodetector 3, the signal light L2 propagates through a space, except within a section in which the signal light L2 passes through the optical member 4.

Furthermore, the optical member 4 may be disposed at a position where the distance between the subject portion Mp0 in the target part Ob and the diaphragm 22 of the light emission unit 2 is internally divided to a_{IN}:b_{IN}, where 1/f_{IN} = 1/a_{IN} + 1/b_{IN} holds true, and f_{IN} is the focal length of the optical member 4.

Note that, in the device 1, an optical design is adopted so that the specific region to which the above-described laser beam L1 is condensed is set to a depth corresponding to a blood-vessel region located inward of the epidermis in the subject portion Mp0, or, that is, a portion of the living body located inward of the epidermis, such as the dermis for example. In the present description, the region in the subject portion Mp0 to which the laser beam L1 is condensed is also referred to as the laser-beam condensing region. Here, for example, the blood-vessel region is located in a portion of the living body that is located inward of the epidermis (also referred to hereinafter as "portion of the living body inwards of the surface of the skin" or "inwards portion of the living body"), such as the dermis for example. For example, the depth corresponding to the blood-vessel region may be 0.5-3.5 mm, inclusive, and may be 1.5 mm, for example.

The incident angle of the laser beam L1 on the subject portion Mp0 is determined by the angle of the optical path of the laser beam L1 from the light emission unit 2 to the laser-beam condensing region with respect to the normal to the portion of the surface of the skin of the target part Ob of the subject person on which the laser beam L1 is incident. In the present embodiment, the incident angle may be set to 0 degrees or more and less than 90 degrees, more preferably 0-80 degrees, inclusive, and further preferably 0-65 degrees, inclusive, for example.

The light-receiving angle of the optical member 4 relating to the signal light L2 is determined by the angle of the optical axis of the signal light L2 passing through the center of the optical member 4 with respect to the normal to the portion of the surface of the skin of the subject portion Mp0 from which the signal light L2 is emitted. The angle of the optical axis passing through the center of the optical member 4 is equal to the installation angle of the photodetector 3 relative to the surface of the skin of the target part Ob of the subject person, and may be set to more than -90 degrees and less than 90 degrees, more preferably -80 degrees to 80 degrees, inclusive, and further preferably -10 degrees to 25 degrees, inclusive, for example, in the present embodiment.

### (Optical-Member Movable Unit 5)

The optical-member movable unit 5 (also referred to hereinafter as "movable unit 5") includes the optical-member holder 51, which holds the optical member 4, and a position changing unit 52 (linear transportation mechanism) that is connected to the optical-member holder 51 and includes a linear transportation mechanism that can reversibly move the optical member 4 along the optical path from the beam splitter 32 to the subject portion Mp0, i.e., a shared optical path shared by the laser beam L1 and the signal light L2. Specifically, the position changing unit 52 has the function of simultaneously changing the distance of the optical member 4 from the image source (diaphragm 22) of the light emission unit 2 and the subject portion Mp0 and the optical distance of the optical member 4 from the light emission unit 2 and the photodetector 3 by moving the optical member 4 along the shared optical path of the laser beam L1 and the signal light L2 from the subject portion Mp0 to the beam splitter 32.

Thus, by moving the optical member 4 along the above-described shared optical path by the position changing unit 52 in a state in which the laser beam L1 is being emitted from the light emission unit 2, the position of the optical member 4 in a direction parallel to the shared optical path of the laser beam L1 and the signal light L2 can be adjusted so that an image at the depth corresponding to the blood-vessel region in the subject portion Mp0 is transferred as an image of equivalent size onto the light receiving surface 31a of the photodetector 3.

Furthermore, the position changing unit 52 may be capable of reversibly moving the infrared sensor 31 along the optical path of the signal light L2, together with the optical member 4.

A general-purpose linear transportation mechanism such as a linear motor, a lead screw, or the like can be used as the position changing unit 52. Alternatively, a reflection mechanism (mirror) including a micro-electromechanical systems (MEMS) actuator in which a piezoelectric element is used, etc., may be inserted into the laser optical path to change the laser optical path.

The movable mechanism 5 is electrically connected to the later-described controller 9. The movable unit 5 is driven by a control signal from the controller 9 to transport the optical member 4 to a predetermined position along the above-described shared optical path, and also outputs, to the controller 9, position information in the direction.

### (Controller 9)

The controller 9 is electrically connected to the light emission unit 2, the photodetector 3, and the movable unit 5. The controller 9 is circuitry that drives the laser light source 21 of the light emission unit 2 to cause the laser light source 21 to oscillate pulsed pump light, and detects the light intensity of the signal light L2 based on an output signal from the photodetector 3 to calculate the concentration of the substance in blood in the blood-vessel region of the subject portion Mp0. Here, output from a photodetector for monitoring may be input to the controller 9, and the controller 9 may normalize the output from the photodetector 3 using the output from the photodetector for monitoring.

Furthermore, the controller 9 is electrically connected to the movable unit 5, and functions as control circuitry that drives the position changing unit 52 of the movable unit 5. That is, the controller 9 adjusts the position of the optical member 4 by outputting a control signal to the movable unit 5 and transporting the optical member 4 as described above. Specifically, in a state in which the laser beam L1 is being emitted from the light emission unit 2 and the light-reception intensity of the signal light L2 is being input from the photodetector 3, the controller 9 searches for a position of the optical member 4 at which the absolute value of the intensity of the signal light L2 or the distribution of the intensity of the signal light L2 on the light receiving surface is in a predetermined state by driving the position changing unit 52 and moving the optical member 4 along the shared optical path of the laser beam L1 and the signal light L2 reversibly, for example, and the controller 9 transports the optical member 4 to the position that is found.

For example, the controller 9 may include a controller (unillustrated) with an internal central processing unit (CPU), and a data storage unit (unillustrated).

The data storage unit includes a volatile memory such as a dynamic random access memory (DRAM), for example, and a nonvolatile memory such as a hard disk, for example. An output signal acquired by the photodetector 3 is transmitted and stored to the data storage unit. Furthermore, the position information of the optical member 4 in the direction parallel to the optical path output from the movable unit 5 is transmitted and stored to the data storage unit. Furthermore, the concentration of the substance in blood calculated by the controller may be stored to the data storage unit. Furthermore, the data storage unit stores therein a program, etc., that are necessary for the execution of the functions of the device 1, and also has a function as a temporary storage area for temporarily storing results of calculation by the controller.

The CPU realizes the functions of the device 1 by reading out and executing the program from the data storage unit. Thus, the controller 9 executes the later-described substance-in-blood concentration measurement processing based on a predetermined program and executes the above-described processing, such as the transportation of the photodetector 3, the emission of the laser beam L1 from the light emission unit 2, and the calculation of the concentration of the substance in blood based on a signal from the photodetector 3.

### <Operation of Substance-In-Blood Concentration Measurement Device 1>

Next, an outline of the operation of the device 1 will be described.

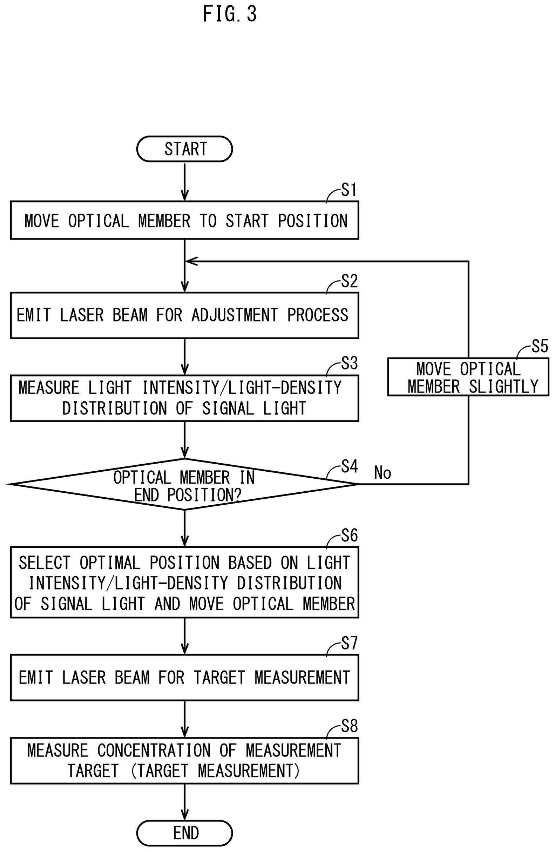

FIG. 3 is a flowchart illustrating a substance-in-blood measurement operation by the substance-in-blood concentration measurement device 1.

In FIG. 3, steps S1 to S6 are steps in which, as the adjustment process, the optical member 4 is moved along the above-described shared optical path by the position changing unit 52 in a state in which the laser beam L1 is being emitted from the light emission unit 2, whereby the position of the optical member 4 in a direction parallel to the shared optical path of the laser beam L1 and the signal light L2 is adjusted so that an image at the depth corresponding to the blood-vessel region in the subject portion Mp0 is transferred as an image of equivalent size onto the light receiving surface 31a of the photodetector 3.

Steps S7 and S8 are steps for the target measurement, in which the concentration of the measurement-target substance is measured in a state in which the laser-beam condensing region is included in the blood-vessel region in the subject portion Mp0.

First, based on a control signal, the controller 9 drives the position changing unit 52 of the movable unit 5 to move the optical member 4 to a start position (step S1), emits the laser beam for the adjustment process from the light emission unit 2 (step S2), and measures the light intensity of the signal light L2 and/or the distribution of the light intensity of the signal light L2 by means of the photodetector 3 (step S3). In this case, the light emission unit 2 and the photodetector 3 constitute a distance-related-information measuring unit that measures information that changes based on a distance to the target part Ob from a specific position on optical paths of the laser beam L1 and the signal light L2. Here, the laser beam for the adjustment process emitted from the light emission unit 2 may be light having a wavelength that is the same as or different from the wavelength of the target-measurement laser beam, as long as the laser beam for the adjustment process can be detected by the photodetector 3.

Based on an output signal from the photodetector 3, the controller 9 calculates and stores the absolute value of the light intensity of the signal light L2 and/or the distribution of the light intensity of the signal light L2 on the light receiving surface 31a. At the same time, the controller 9 stores position information of the optical member 4 in the measurement.

Next, it is determined whether or not the optical member 4 is in an end position (step S4). If the optical member 4 is not in the end position (step S4: No), the position of the optical member 4 is moved slightly (step S5), and processing returns to step S2, whereas, if the optical member 4 is in the end position (step S4: Yes), processing proceeds to step S6, it being regarded that the measurement has been performed at all positions of the optical member 4 in the adjustment process. In this state, position information of the optical member 4, and measured data of the light intensity of the signal light L2 and/or the distribution of the light intensity of the signal light L2 at all positions of the optical member 4 have been stored in the controller 9.

In step S6, based on the measurement data at all positions of the optical member 4, the controller 9 selects, as an optimal position, a position of the optical member 4 where the light intensity of the signal light L2 is highest and/or the distribution of the light intensity of the signal light L2 is concentrated in the smallest area. Furthermore, the controller 9 transmits a control signal to the movable unit 5 to drive the position changing unit 52 and move the optical member 4 to the optimal position. It can be inferred that the laser-beam condensing region is included in the blood-vessel region in the subject portion Mp0 at the optimal position.

Next, the concentration of the measurement-target substance is measured in steps S7 and S8. Specifically, the laser beam for target measurement is condensed and emitted from the light emission unit 2 to the specific region in the subject portion Mp0 (step S7), a measurement of the concentration of the measurement target in the laser-beam condensing region is performed by means of the photodetector 3 to output a measurement result (step S8), and processing is terminated.

### <Section Summary>

As described above, a substance-in-blood concentration measurement device 1 according to Embodiment 1 is a substance-in-blood concentration measurement device 1 that measures a concentration of a substance in blood included in the blood in a subject portion Mp0 in a target part Ob of a subject person, the substance-in-blood concentration measurement device 1 being characterized by including: a measuring unit (2, 3) that measures information relating to the target part Ob of the subject person; a light emission unit 2 that emits a laser beam L1 to a specific region in the subject portion in the target part Ob; a photodetector 3 that receives signal light L2 reflected from the subject person based on the laser beam L1 and detects an intensity of the signal light L2; an optical member 4 that condenses the laser beam L1 to the specific region in the subject portion Mp0 in the target part Ob, and forms, on the photodetector 3, a focused image of the signal light L2 from a region to which the laser beam L1 is condensed in the subject portion Mp0; a movable unit 5 that changes a state of the optical member 4; and a controller 9 that, based on the intensity of the signal light L2, obtains a concentration of the substance in blood in the region to which the laser beam L1 is condensed, wherein the movable unit 5 changes the state of the optical member 4 based on the information relating to the target part Ob.

Furthermore, the measuring unit may include a distance-related-information measuring unit (2, 3) that measures, as the information relating to the target part Ob, information that changes based on a distance to the target part Ob from a specific position on optical paths of the laser beam L1 and the signal light L2. The optical member 4 may be present on an optical path shared by the laser beam L1 and the signal light L2. Based on the information measured by the distance-related-information measuring unit (2, 3), the movable unit 5 may transfer the optical member 4 along the shared optical path to a position where the region to which the laser beam L1 is condensed is capable of matching the specific region in the subject portion Mp0 in the target part Ob, and where a focused image of the signal light L2 from the region to which the laser beam L1 is condensed can be formed on the photodetector 3, whereafter the light emission unit 2 may emit the laser beam L1, the photodetector 3 may receive the signal light L2 based on the laser beam L1 and detect the intensity of the signal light L2, and the controller 9 may measure the concentration of the substance in blood based on the intensity of the signal light L2.

According to such structures, because the device 1 includes therein a function for focusing the light emission unit and the photodetector to a portion in the target part Ob of the subject person, the concentration of the substance in blood can be measured by an operator emitting the laser beam L1 to the target part Ob of the subject person in a state in which the distal-end portion 1a of the device 1 is directed toward the direction of the target part Ob of the subject person.

Thus, according to the device 1, the concentration of the substance in blood can be measured more simply and stably by taking advantage of the characteristics of optical measurement and using, as the measurement-target part, parts of the body of a subject person where the skin is exposed, which include not only the fingers but also other parts such as the forehead portion of the subject person, for example.

Note that, with the device 1, measurement can also be performed using, as the target part Ob, other portions of the body of the subject person where the skin is exposed, besides the forehead,

### <Modifications>

Up to this point, a specific structure of the device 1 has been described taking Embodiment 1 as an example, but the present disclosure is not limited by the above embodiment in any way, except for essential characteristic constituent elements thereof.

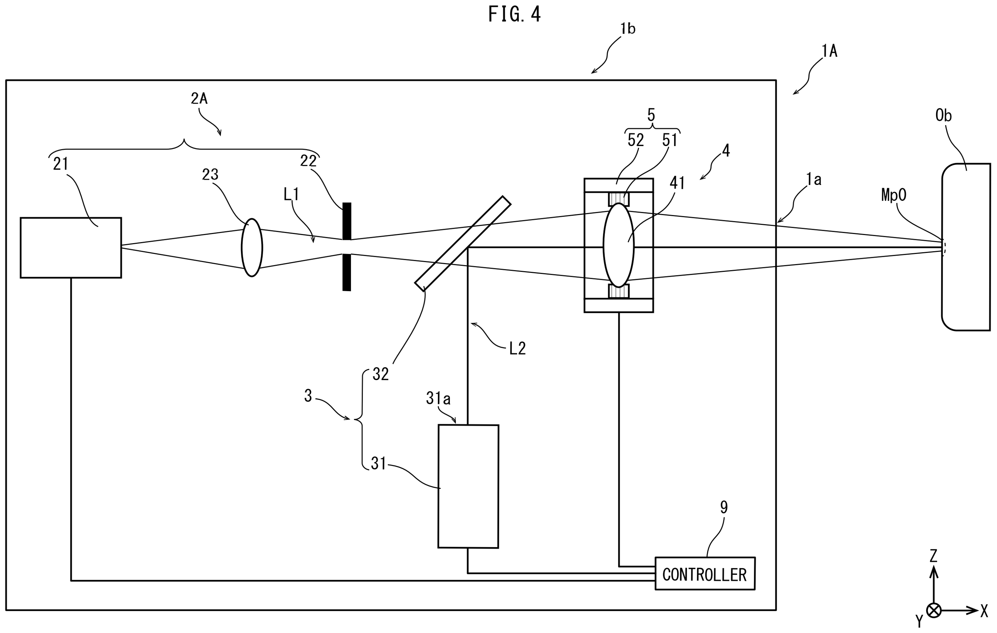

In the following, modifications of the device 1 will be described with reference to FIGS. 4 to 7.

(1) FIG. 4 is a diagram illustrating a structure of a substance-in-blood concentration measurement device 1A (also referred to hereinafter as "device 1A"), in which the light emission unit 2 in the device 1 has been modified into a different structure. As illustrated in FIG. 4, in the device 1A, a light emission unit 2A includes, between the laser light source 21 and the diaphragm 22, a condenser lens 23 the focal point of which is adjusted to the opening of the diaphragm 22.

According to the device 1A, by temporarily condensing the laser beam L1 emitted from the laser light source 21 to the opening of the diaphragm 22, a small original image with a small loss in light amount can be formed, and a focused image of this original image can be transferred, by the optical member 4, to the specific region in the subject portion Mp0 to which the laser beam L1 is to be condensed. Thus, the device 1A can reduce the loss in light amount (light energy) compared to the device 1.

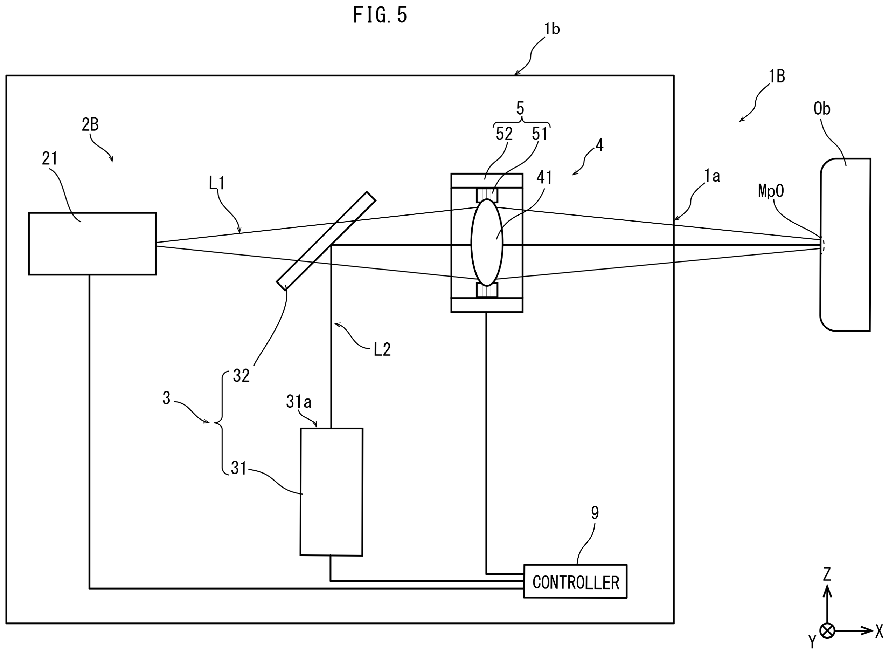

(2) FIG. 5 is a diagram illustrating a structure of a substance-in-blood concentration measurement device 1B (also referred to hereinafter as "device 1B"), in which the light emission unit 2 in the device 1 has been modified into a different structure. As illustrated in FIG. 5, in the device 1B, a light emission unit 2B emits an original image from a light emission point of the laser light source 21 without using the diaphragm 22, and a focused image of this original image is transferred, by the optical member 4, to the laser-beam condensing region in the subject portion Mp0, to which the laser beam L1 is to be condensed. According to the device 1B, the image source (i.e., the diaphragm 22 in the device 1) is replaced with a light emission point of the laser light source 21 having the same size. Thus, the diaphragm 22 can be reduced and device structure can be simplified compared to the device 1 while ensuring that a focused image can be transferred to the specific region in the subject portion Mp0.

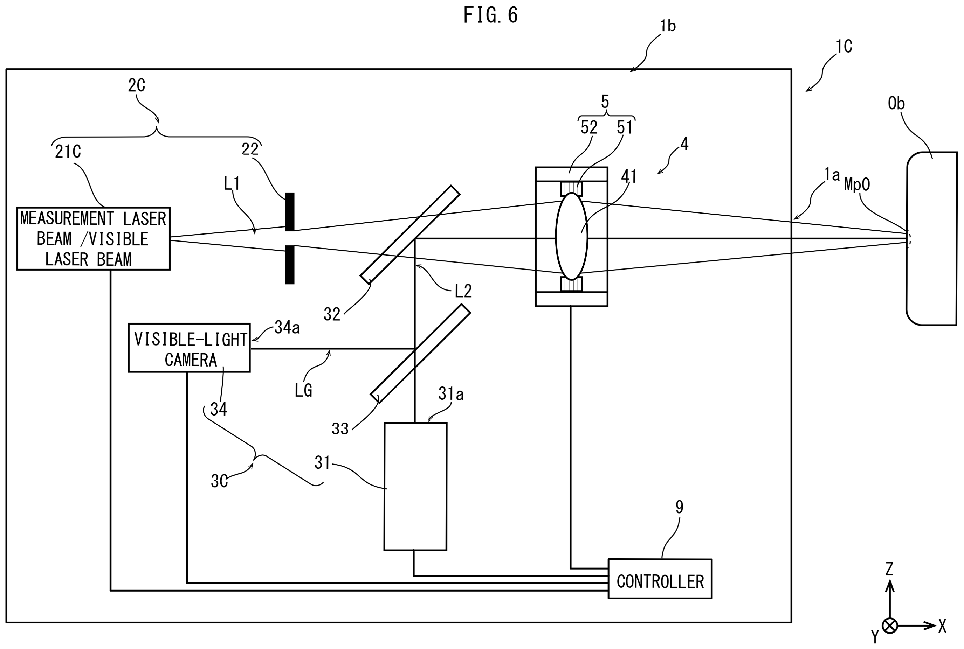

(3) FIG. 6 is a diagram illustrating a structure of a substance-in-blood concentration measurement device 1C (also referred to hereinafter as "device 1C"), in which the light emission unit 2 and the photodetector 3 in the device 1 have been modified into different structures. As illustrated in FIG. 6, in the device 1C, a light emission unit 2C can selectively emit the target-measurement laser beam L1 and a visible laser beam LG used as the guide light in the adjustment process.

Furthermore, in the device 1C, a photodetector 3C includes a dichroic mirror 33 that is formed from a germanium plate or the like, for example, on the optical path from the beam splitter 32 to the infrared sensor 31. Furthermore, the photodetector 3C includes a visible-light camera 34 downstream of the dichroic mirror 33, and the optical path distance (geometric distance) from the optical member 4 to a light receiving surface 34a of the visible-light camera 34 is set to be equivalent to the distance from the optical member 4 to the light receiving surface 31a of the infrared sensor 31.

Thus, reflected light from the target part Ob based on the visible laser beam LG can be received by means of the visible-light camera 34.

According to such a device 1C, by selectively emitting the laser beam L1 and the guide light (i.e., the visible laser beam LG) in the target measurement and the adjustment process, respectively, the light intensity and/or the distribution of the light intensity of the reflected light from the target part Ob based on the guide light (i.e., the visible laser beam LG) can be measured by means of the visible-light camera 34 in the adjustment process.

Thus, based on visible light having higher visibility, a position of the optical member 4 where the light intensity is highest and/or the distribution of the light intensity is concentrated in the smallest area can be selected as the optimal position in the adjustment process. Furthermore, in this case, the correction of a minute positioning error occurring due to the difference in wavelength from the target-measurement laser beam may be performed on the optimal position selected in the adjustment process when the position of the optical member 4 is set in the target measurement.

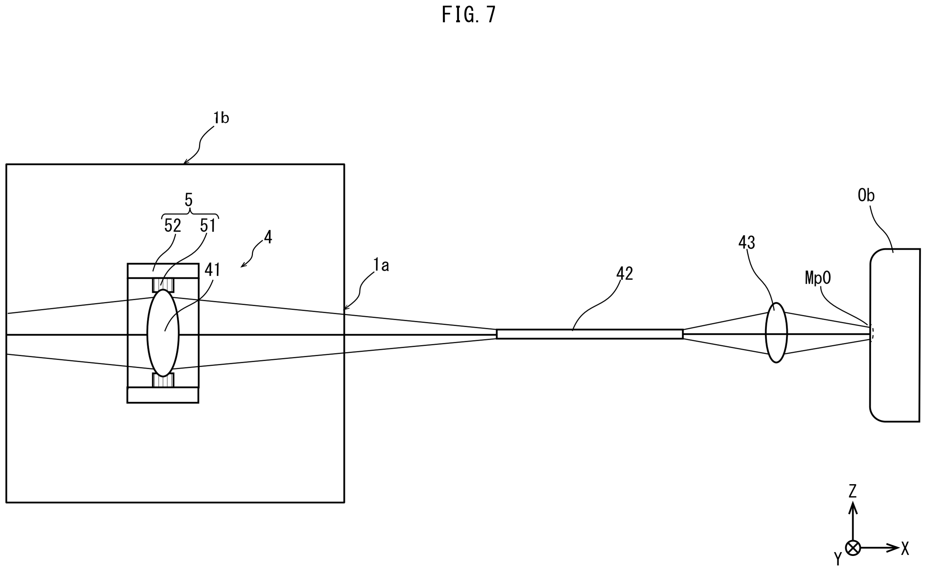

(4) FIG. 7 is a diagram illustrating a different embodiment of the structure from the optical member 4 to the target part Ob in the devices 1, 1A, 1B, and 1C. In this embodiment, an optical fiber 42 and a condenser lens 43 are provided in the distal-end portion of the device, i.e., the optical path from the optical member 4 to the target part Ob. In this structure, light condensed by the optical member 4 is guided by the optical fiber 42, and is condensed again by the condenser lens 43 to the specific region in the subject portion Mp0.

According to such an embodiment of the devices 1, 1A, 1B, and 1C, by inserting the distal-end portion of the device into the body cavity via a laparoscope, an endoscope, or the like and measuring the concentration of the substance in blood, the concentration of the substance in blood in the body can be measured during surgery. Furthermore, a simple, portable device can be realized.

(5) In the adjustment process of the device 1, information that changes based on a distance to the target part from a specific position on optical paths of the laser beam and the signal light is measured as the information relating to the target part. That is, in the device 1, in a state in which the laser beam L1 is being emitted from the light emission unit 2 and the light-reception intensity of the signal light L2 is being input from the photodetector 3, a search is performed for a position of the optical member 4 at which the absolute value of the intensity of the signal light L2 or the distribution of the intensity of the signal light L2 on the light receiving surface is in a predetermined state by driving the position changing unit 52 and moving the optical member 4 along an optical path, and the optical member 4 is transported to the position that is found.

However, the measuring unit may measure, as the information relating to the target part, a distance to the target part Ob from a specific position on optical paths of the laser beam L1 and the signal light L2, and, based on the distance measured by the measuring unit, the movable unit 5 may transport the optical member 4 along the shared optical path to a position where the region to which the laser beam L1 is condensed is capable of matching the specific region in the subject portion Mp0 in the target part Ob, and where a focused image of the signal light L2 from the region to which the laser beam is condensed can be formed on the photodetector 3.

In this case, the measurement of the distance to the target part Ob may be performed by measuring the time until signal light L2 based on the emission of the laser beam L1 or the visible laser beam by the light emission unit 2 is received by the photodetector 3 or the visible-light camera 34 after the laser beam L1 or the visible laser beam is emitted. Alternatively, the distance to the target part Ob may be measured using a laser distance measuring unit (distance measurement device) separately from such elements.

Furthermore, the optical member 4 may be transported as follows. That is, an optimal position identified based on distance data acquired in advance may be stored in advance in the controller 9, and the optical member 4 may be transported to the optimal position based on output from a distance measuring unit.

### <<Embodiment 2>>

### <Overall Structure>

Measurement using the device 1 according to above-described Embodiment 1 is performed by the operator emitting the laser beam L1 to the surface of the forehead of the subject person in a state in which the distal-end portion 1a of the device 1 is directed toward the direction of the target part Ob of the subject person. In contrast, a substance-in-blood concentration measurement device 1D (also referred to hereinafter as "device 1D") according to Embodiment 2 differs from the device 1 in that the substance-in-blood concentration measurement device 1D includes therein a function for directing a light emission unit and a photodetector toward the direction of the target part Ob of the subject person.

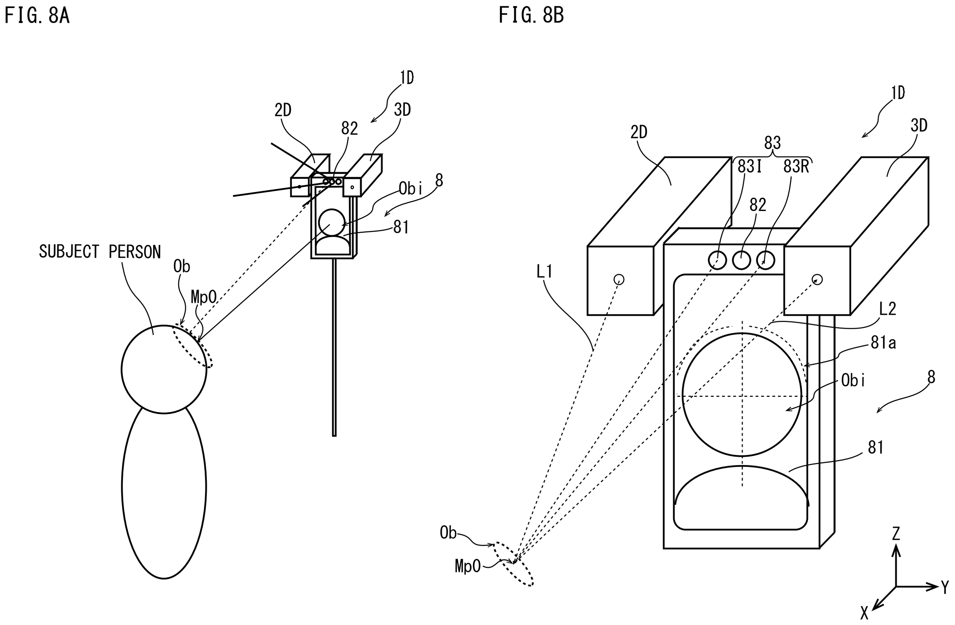

FIG. 8A is a schematic diagram illustrating a state of the device 1D during measurement.

With the device 1D, the concentration of the substance in blood is measured in a state in which the subject person is facing the front of the device 1D and an image Obi of the target part Ob of the subject person captured by a camera 82 is being displayed on a display unit 81 of a main-body portion 8 at the center of the device 1D.

In the following, a structure of the device 1D will be described with reference to the drawings.

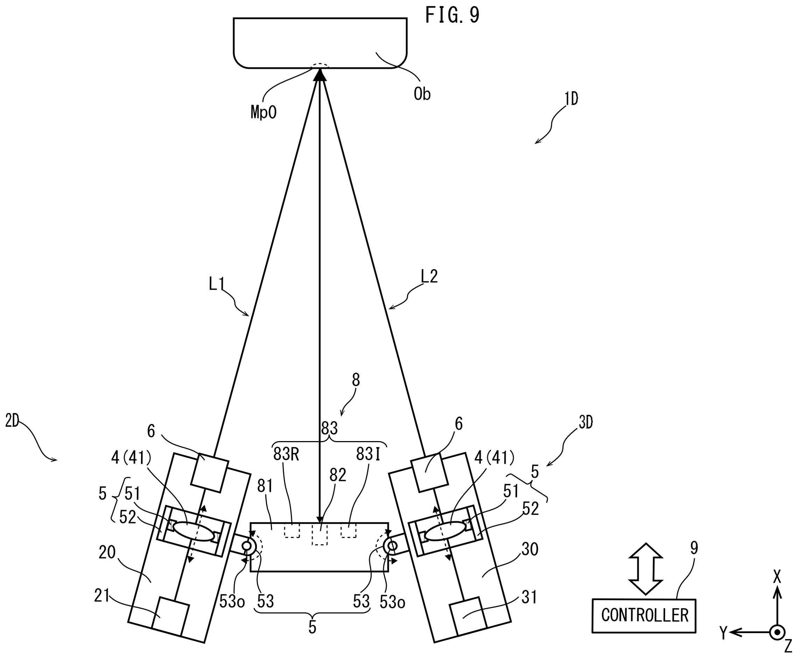

FIG. 8B is a perspective view of the device 1D. FIG. 9 is a horizontal cross-sectional view illustrating the structure of the device 1D.

The device 1D includes the main-body portion 8, a light emission unit 2D and a photodetector 3D that are each connected to a side surface of the main-body portion 8 via an angle changing unit 53, and a controller 9. In the following, structures of the elements of the device 1D will be described. Among the elements, constituent elements that are the same as those in the device 1 are provided with the same reference numerals, and description of the individual structures and functions thereof is omitted.

### <Structures of Elements>

The main-body portion 8 constitutes the main-body unit of the device 1D, and includes, on the front surface thereof, the display unit 81, the camera 82 (position measurement device), and an optical distance measuring unit 83 (distance measurement device) that includes a light emission unit 83l and a light receiving unit 83R. The camera 82 is activated based on sensing by the distance measuring unit 83, and an image captured by the camera 82 is displayed in real time on the display unit 81.

Via an angle changing unit 53 constituting a movable unit 5, the light emission unit 2D is supported on the right side surface of the main-body portion 8 in a front view so as to be rotatable about a shaft 53o. Furthermore, the angle changing unit 53 may be a two-axis angle changing unit that can also rotate within a horizontal plane about a shaft that is perpendicular to the optical path of the laser beam L1.

The light emission unit 2D includes a laser light source 21 inside a housing 20. The laser light source 21, a condenser lens 41 constituting an optical member 4, and a position changing unit 52 constituting the movable unit 5 are installed inside the housing 20 of the light emission unit 2D, and the target-measurement laser beam L1 can be condensed and emitted to the target part Ob of the subject person present in a position located at a predetermined distance. The light emission unit 2D includes a visible laser light source 6 that can emit, in the same direction as the target-measurement laser beam L1, a visible laser beam LG to be used as guide light in an angle adjustment process.

Similarly, via an angle changing unit 53 constituting a movable unit 5, the photodetector 3D is supported on the left side surface of the main-body portion 8 in the front view so as to be rotatable about a shaft 53o. Furthermore, the angle changing unit 53 may be a two-axis angle changing unit that can also rotate within a horizontal plane about a shaft that is perpendicular to the optical path of signal light L2.

The photodetector 3D includes an infrared sensor 31 inside a housing 30. The infrared sensor 31, a condenser lens 41 that constitutes an optical member 4 and that functions as an imaging lens, and a position changing unit 52 constituting the movable unit 5 are installed inside the housing 30 of the photodetector 3D, and the signal light L2 emitted from the laser-beam condensing region in the target part Ob of the subject person based on the emitted laser beam L1 is received and the intensity thereof is detected. Furthermore, the photodetector 3D includes a visible laser light source 6 that can emit, in the same direction as the target-measurement laser beam L1, a visible laser beam LG to be used as guide light in the angle adjustment process.

The controller 9 is electrically connected to the light emission unit 2D, the photodetector 3D, and the movable units 5 (position changing units 52 and angle changing units 53), and controls such elements by transmitting control signals.

### <Operation of Device 1D>

Next, an outline of the operation of the device 1D will be described.

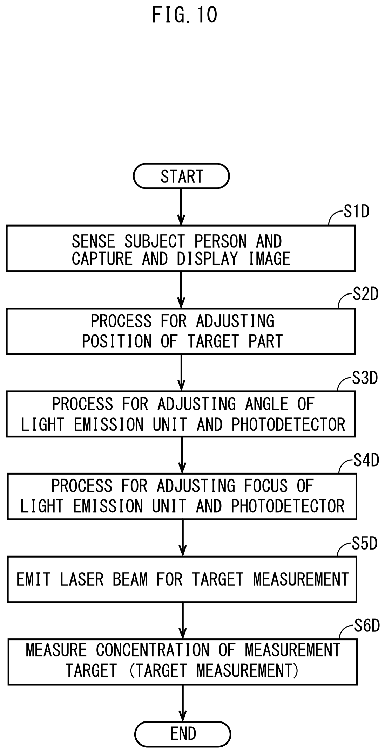

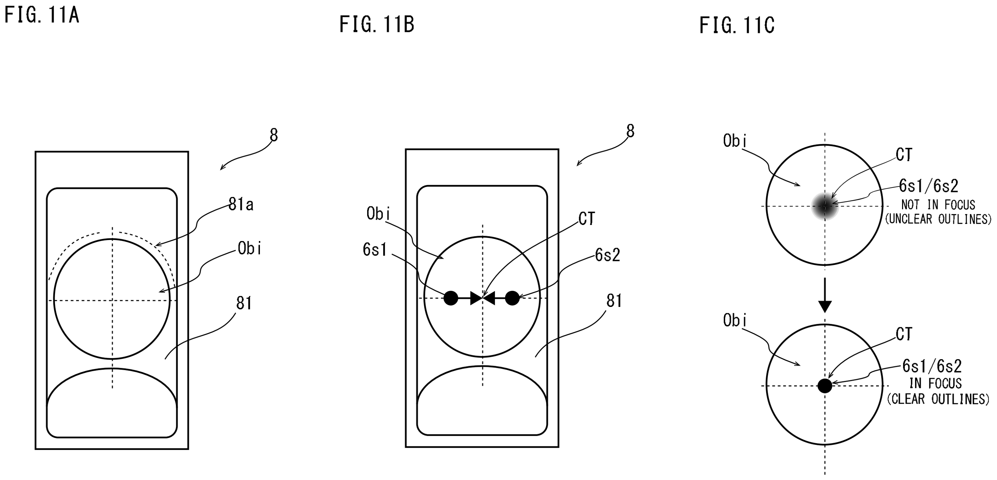

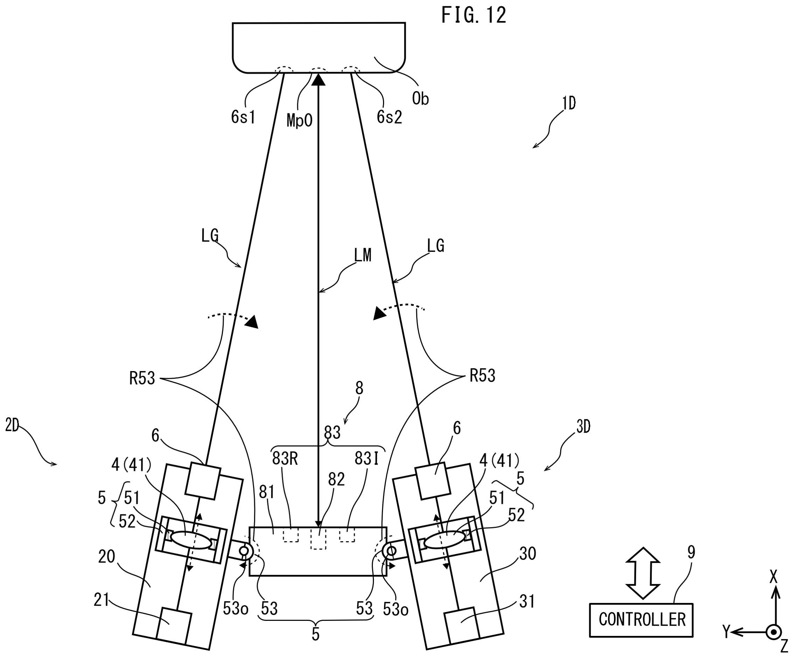

FIG. 10 is a flowchart illustrating a substance-in-blood measurement operation by the device 1D. FIGS. 11A to 11C, and FIG. 12 are explanatory diagrams for explaining an adjustment operation by the device 1D.

In steps S1D to S4D, the position of the subject person, and the angle and focus of the light emission unit 2D and the photodetector 3D are adjusted.

First, when the subject person faces the front of the device 1D, the camera 82 is activated based on sensing by the distance measuring unit 83, and an image Obi of the target part Ob of the subject person that is captured by the camera 82 is displayed on the display unit 81 of the main-body portion 8 (step S1D).

In this state, as illustrated in FIG. 11A, the subject person adjusts the position of his/her target part Ob relative to the device 1D so that the image Obi fits inside a predetermined area 81a on the display unit 81 (step S2D).

Next, the angle adjustment process of the light emission unit 2D and the photodetector 3D is performed (step S3D).

In the angle adjustment process, first, the visible laser light sources 6 installed in the light emission unit 2D and the photodetector 3D emit visible laser beams LG as guide light, and the camera 82 captures images 6s1 and 6s2 of the visible laser beams LG emitted to the target part Ob and outputs the images 6s1 and 6s2 to the controller 9 (the images may be displayed on the display unit 81).

As illustrated in FIG. 11B, the controller 9 calculates the optimal angles of the light emission unit 2D and the photodetector 3D for ensuring that the two images 6s1 and 6s2 from the visible laser light sources 6 emitted from the light emission unit 2D and the photodetector 3D match a target position CT in the left-right direction or the up-down and left-right directions, and outputs the result of the calculation to the angle changing units 53.

Next, as illustrated in FIG. 12, the angle changing unit 53 supporting the light emission unit 2D changes the angle of the light emission unit 2D (arrow R53 in FIG. 12) to the optimal angle based on a control signal, and the angle changing unit 53 supporting the photodetector 3D changes the angle of the photodetector 3D (arrow R53 in FIG. 12) to the optimal angle based on a control signal. Here, the change in angle may be a change in angle about a single axis (vertical-direction axis), or a change in angles about two axes (vertical-direction axis that is perpendicular to the optical paths within a horizontal plane and horizontal-direction axis that is perpendicular to the optical paths within a vertical plane).

Next, a focus adjustment process of the light emission unit 2D and the photodetector 3D is performed (step S4D).

In the focus adjustment process, a distance-measurement laser beam LM is emitted by the distance measuring unit 83 to measure the distance to the target part Ob, and, based on the distance from the device 1D to the target part Ob measured by the distance measuring unit 83: the movable unit 5 (position changing unit 52) of the light emission unit 2D transports the optical member 4 to a position where the region to which the laser beam L1 is condensed is capable of matching the specific region in the subject portion Mp0 of the target part Ob; and the movable unit 5 (position changing unit 52) of the photodetector 3D transports the optical member 4 to a position where a focused image of the signal light L2 from the region to which the laser beam L1 is condensed can be formed on the photodetector 3D.

Alternatively, a different adjustment method illustrated in FIG. 11C may be used in the focus adjustment process. Specifically, the images 6s1 and 6s2 of the visible laser beams LG emitted to the target part Ob from the visible laser light sources 6 installed in the light emission unit 2D and the photodetector 3D are captured by the camera 82, and it is determined from the images 6s1 and 6s2 whether or not the images 6s1 and 6s2 have clear outlines. Furthermore, a search for positions (focal positions) of the optical members 4 where the outlines of the left and right images 6s1 and 6s2 become clear is performed while slightly moving the optical members 4 in the optical-path direction by the movable units 5 (position changing units 52), and the optical members 4 of the light emission unit 2D and the photodetector 3D are transported to the respective positions.

Next, the concentration of the measurement-target substance is measured in steps S5D and S6D. Specifically, the laser beam for target measurement is condensed and emitted from the light emission unit 2D to the specific region in the subject portion Mp0 (step S5D), a measurement of the concentration of the measurement target in the laser-beam condensing region is performed by means of the photodetector 3D to output a measurement result (step S6D), and processing is terminated.

### <Section Summary>

As described above, in a substance-in-blood concentration measurement device 1D according to Embodiment 2, which measures a concentration of a substance in blood included in the blood in a subject portion Mp0 in a target part Ob of a subject person, the substance-in-blood concentration measurement device 1D includes: a measuring unit (83, 6, 82) that measures information relating to the target part Ob of the subject person; a light emission unit 2 that emits a laser beam L1 to a specific region in the subject portion in the target part Ob; a photodetector 3 that receives signal light L2 reflected from the subject person based on the laser beam L1 and detects an intensity of the signal light L2; an optical member 4 that condenses the laser beam L1 to the specific region in the subject portion Mp0 in the target part Ob, and forms, on the photodetector 3, a focused image of the signal light L2 from a region to which the laser beam L1 is condensed in the subject portion Mp0; a movable unit 5 that changes a state of the optical member 4; and a controller 9 that, based on the intensity of the signal light L2, obtains a concentration of the substance in blood in the region to which the laser beam L1 is condensed. The movable unit 5 changes the state of the optical member 4 based on the information relating to the target part Ob.

That is, the substance-in-blood concentration measurement device 1D according to Embodiment 2 is characterized in that the measuring unit includes a distance measuring unit 83 that measures, as the information relating to the target part Ob, a distance to the target part Ob from a specific position on optical paths of the laser beam L1 and the signal light L2. The optical member 4 includes: a first optical member 4 that condenses the laser beam L1 to the specific region in the subject portion Mp0 in the target part Ob; and a second optical member 4 that forms, on the photodetector 3, a focused image of the signal light L2 from the region to which the laser beam L1 is condensed in the subject portion Mp0. The movable unit 5 includes: a first position changing unit 52 that controls a position of the first optical member 4 on the optical path of the laser beam L1; and a second position changing unit 52 that controls a position of the second optical member 4 on the optical path of the signal light L2, the first position changing unit 52 and the second position changing unit 52 being respectively disposed in a housing 20 and a housing 30. Based on the distance measured by the distance measuring unit 83, the first position changing unit 52 disposed in the housing 20 transfers the first optical member 4 to a position where the region to which the laser beam L1 is condensed is capable of matching the specific region in the subject portion Mp0 in the target part Ob, and, based on the distance measured by the distance measuring unit 83, the second position changing unit 52 disposed in the housing 30 transfers the second optical member 4 to a position where a focused image of the signal light L2 from the region to which the laser beam L1 is condensed can be formed on the photodetector 3, whereafter the light emission unit 2 emits the laser beam L1, the photodetector 3 receives the signal light L2 based on the laser beam L1 and detects the intensity of the signal light L2, and the controller 9 measures the concentration of the substance in blood based on the intensity of the signal light L2.

Furthermore, in the device 1D, the measuring unit may include a position measuring unit (visible laser light sources 6 and camera 82) that measures, as the information relating to the target part Ob, a position of the target part Ob relative to a specific position on optical paths of the laser beam L1 and the signal light L2. The optical member 4 may include: a first optical member 4 that is positioned on the optical path of the laser beam L1 and that condenses the laser beam L1 to the specific region in the subject portion Mp0 in the target part Ob; and a second optical member 4 that is positioned on the optical path of the signal light L2 and that forms, on the photodetector 3, a focused image of the signal light L2 from the region to which the laser beam L1 is condensed in the subject portion Mp0 in the target part Ob. The movable unit 5 may include: a first angle changing unit 53 that changes an emission angle of the first optical member 4; and a second angle changing unit 53 that changes a light-receiving angle of the second optical member 4, the first angle changing unit 53 and the second angle changing unit 53 being respectively disposed in the housing 20 and the housing 30. Based on the position measured by the position measuring unit (6, 82, 9), the first angle changing unit 53 disposed in the housing 20 may change the emission angle of the first optical member 4 to a direction in which the region to which the laser beam L1 is condensed is capable of matching the specific region in the subject portion Mp0 in the target part Ob, and, based on the positioned measured by the position measuring unit (6, 82, 9), the second angle changing unit 53 disposed in the housing 30 may change the light-receiving angle of the second optical member 4 to a direction in which a focused image of the signal light L2 from the region to which the laser beam L1 is condensed can be formed on the photodetector 3, whereafter the light emission unit 2 may emit the laser beam L1, the photodetector 3 may receive the signal light L2 based on the laser beam L1 and detect the intensity of the signal light L2, and the controller 9 may measure the concentration of the substance in blood based on the intensity of the signal light L2.

According to such structures, the device 1D includes therein a function for focusing the light emission unit 2D and the photodetector 3D to a portion in the target part Ob of the subject person and a function for directing the light emission unit 2D and the photodetector 3D toward the direction of the target part Ob of the subject person. Thus, the concentration of the substance in blood is measured in a state in which the subject person is facing the front of the device 1D and an image Obi of the target part Ob of the subject person captured by the camera 82 is being displayed on the display unit 81 of the main-body portion 8.

Thus, the concentration of the substance in blood can be measured more simply and stably by taking advantage of the characteristics of optical measurement and using, as the measurement-target part, not only the fingers but also the face, etc., of the subject person.

In the device 1D according to Embodiment 2, an embodiment in which the angle changing units 53 change the angles of the light emission unit 2D and the photodetector 3D has been described as an example. However, as the "angle changing units", a reflection mechanism (mirror) including a MEMS actuator in which a piezoelectric element is used, etc., may be inserted into the laser optical path to change the laser optical path and thereby change the emission angle of the light emission unit 2D and the light-receiving angle of the photodetector 3D.

In this case, in place of moving the entire light emission unit 2D and the entire photodetector 3D, the "angle changing units" may change the emission angle of the light emission unit 2D and the light-receiving angle of the photodetector 3D by driving the optical members 4 and changing the laser optical path by using a reflection mechanism (mirror) including a motor or a MEMS actuator in which a piezoelectric element is used, etc. Here, the optical members 4 may be driven so as to rotate within a vertical plane and/or within a horizontal plane about an axis perpendicular to the optical paths, or may be driven to translate within a horizontal plane about an axis perpendicular to the optical paths.

Furthermore, in the device 1D, the light emission unit 2D, the photodetector 3D, the movable units 5 (position changing units 52 and angle changing units 53), the main-body portion 8, and the controller 9 may be installed inside one housing.

### <<Embodiment 3>>

In the device 1 according to above-described Embodiment 1, an example was described of a structure in which measurement is performed by the operator emitting the laser beam L1 to the surface of the forehead of the subject person in a state in which the distal-end portion 1a of the device 1 is directed toward the direction of the target part Ob of the subject person. Furthermore, in the device 1D according to Embodiment 2, an example was described of a structure in which, when the subject person himself/herself adjusts the position of the target part Ob relative to the device 1D in a state in which the subject person is facing the front of the device 1D and an image Obi of the target part Ob is being displayed on the display unit 81, operations for adjusting the focus and angles of the light emission unit 2D and the photodetector 3D are performed, and the concentration of the substance in blood is measured.

In contrast, substance-in-blood concentration measurement devices 1E to 1I according to Embodiment 3 differ from the devices according to Embodiments 1 and 2 in that the substance-in-blood concentration measurement devices 1E to 1I have the function of measuring the concentration of the substance in blood by emitting the laser beam L1 from a light emission unit to the subject person moving within a predetermined space and receiving, by means of a photodetector, the signal light L2 reflected from the target part Ob.

### (1) Measurement with respect to moving subject person

In the following, a structure of the device 1E according to Embodiment 3 will be described with reference to the drawings.

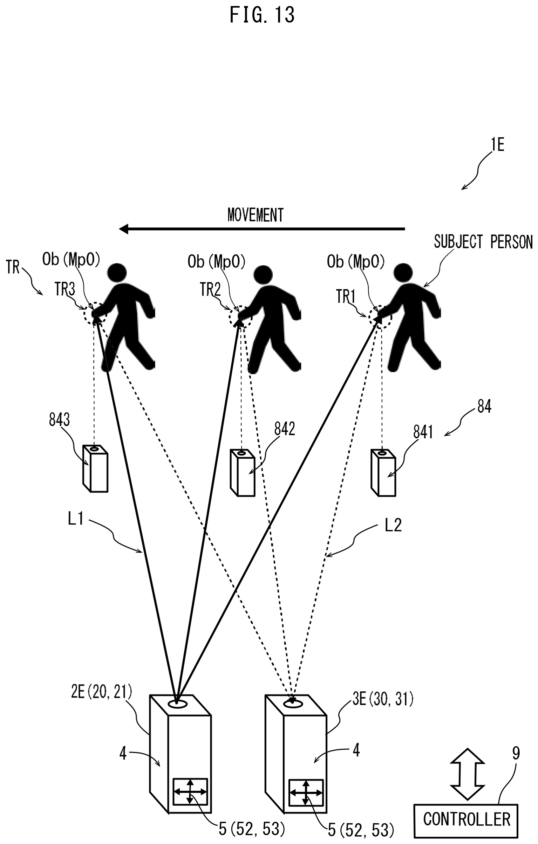

FIG. 13 is a schematic diagram illustrating the structure of the device 1E according to Embodiment 3 (also referred to hereinafter as "device 1E").

As illustrated in FIG. 13, the device 1E includes a light emission unit 2E, a photodetector 3E, optical members 4, position changing units 52, angle changing units 53, a detecting unit 84, and a controller 9 that controls such elements. Constituent elements that are the same as those in the device 1 are provided with the same reference numerals, and description of the individual structures and functions thereof is omitted.

The light emission unit 2E and the photodetector 3E respectively have the same structures as the light emission unit 2D and the photodetector 3D according to Embodiment 2. That is, the light emission unit 2E includes a housing 20 and a laser light source 21. Furthermore, the photodetector 3E includes a housing 30 and an infrared sensor 31.

Furthermore, in the device 1E, an optical member 4, the laser light source 21, and a position changing unit 52 are disposed in the housing 20, for example, similarly to the light emission unit 2D according to Embodiment 2. Furthermore, an optical member 4, the infrared sensor 31, and a position changing unit 52 are disposed in the housing 30, for example, similarly to the photodetector 3D according to Embodiment 2. Furthermore, in the device 1E, an angle changing unit 53 that is the same as that in Embodiment 2 is disposed in each of the housings 20 and 30.

The detecting unit 84 is a sensor for detecting whether or not the target part of the subject person is present within a specific area in a space. The detecting unit 84 is constituted from detecting units 841 to 843. When the subject person passes through a predetermined path, the detecting unit 84 detects whether or not the target part Ob of the subject person is present in specific areas in the space that the target part Ob of the subject person may pass through, namely target areas TR1 to TR3, and outputs the result of the detection to the controller 9.

Based on control signals from the controller 9, the light emission unit 2E and the photodetector 3E actuate the movable units 5 (position changing units 52 and the angle changing units 53) to change the angle and focus of the light emission unit 2E and the photodetector 3E. The angle and focus are changed by the operations of: storing in advance, in the controller 9, optimal angles and optimal optical-member positions corresponding to the target areas TR1 to TR3 identified in advance; and, when a sensing signal from one of the target areas TR1 to TR3 is obtained from the detecting units 841 to 843, changing the angles of the light emission unit 2E and the photodetector 3E to the angles corresponding to the target area TR, and transporting the optical members to the corresponding optimal positions.

Furthermore, the concentration of the substance in blood is measured by emitting the laser beam L1 from the light emission unit 2E to the subject portion Mp0 in the target part Ob of the subject person present in the specific target area TR in the space, and receiving signal light L2 reflected from the subject portion Mp0 in the target part Ob by means of the photodetector 3E.

According to the device 1E having such a structure, in a case such as that in which the subject person passes through a predetermined path, such as a passage inside a building or an athletic track, the concentration of the substance in blood can be measured by emitting the laser beam L1 from the light emission unit 2E to the subject portion Mp0 in the target part Ob of the subject person present in the specific target areas TR1 to TR3 in the space that the target part Ob of the subject person passes through, and receiving the signal light L2 reflected from the subject portion Mp0 in the target part Ob by means of the photodetector 3E.

Thus, by using the device 1E and emitting the laser beam L1 to a part of the subject person exposed from clothes, such as the head, the hands and feet, the arms, the shoulders, the stomach, the back, or the legs, the concentration of a substance in blood, such as glucose or cholesterol for example, in a registered customer may be measured at an entrance or passage to a restaurant, a cafeteria, etc., for example, and items on the menu recommended to the customer may be presented based on the result of the measurement. Alternatively, the device 1E may be used to measure the concentration of a substance in blood, such as glucose or lactic acid for example, in an athlete participating in a track-and-field competition such as a marathon or long-distance running, a swimming competition, etc., and thereby provide support such as pitch control, pace control, and drink provision adapted to the fatigue level and physical state of the athlete.

### (2) Embodiment in which visible-light camera is used as detecting unit

In another embodiment of the device 1E, a target area TR in a space that the target part Ob of the subject person passes through may be identified by capturing and analyzing an image of the subject person using a visible-light camera as a detecting unit.

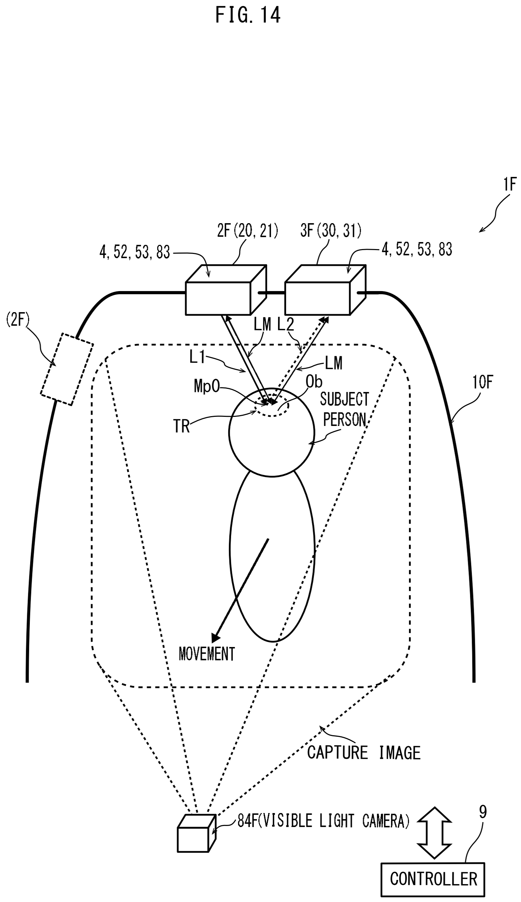

FIG. 14 is a schematic diagram illustrating a structure of a substance-in-blood concentration measurement device 1F according to a modification (also referred to hereinafter as "device 1F").

As illustrated in FIG. 14, the device 1F includes a gate 10F that the subject person passes through, a light emission unit 2F and a photodetector 3F provided to the gate 10F, optical members 4, position changing units 52, angle changing units 53, distance measuring units 83, a detecting unit 84F formed from a visible-light camera, and a controller 9 that controls such elements. The detecting unit 84F may include a plurality of visible-light cameras provided at positions from which images of the subject person can be captured from different angles.

The light emission unit 2F and the photodetector 3F can be provided to desired positions of the gate 10F. The constituent elements of the light emission unit 2F and the photodetector 3F are the same as those of the light emission unit 2D and the photodetector 3D according to Embodiment 2. That is, the light emission unit 2F includes a housing 20 and a laser light source 21. Furthermore, the photodetector 3F includes a housing 30 and an infrared sensor 31.

Furthermore, in the device 1F, an optical member 4, the laser light source 21, and a position changing unit 52 are disposed in the housing 20, for example, similarly to the light emission unit 2D according to Embodiment 2. Furthermore, an optical member 4, the infrared sensor 31, and a position changing unit 52 are disposed in the housing 30, for example, similarly to the photodetector 3D according to Embodiment 2. Furthermore, in the device 1F, an angle changing unit 53 and a distance measuring unit 83 that are the same as those in Embodiment 2 are disposed in each of the housings 20 and 30.

The detecting unit 84F is formed from a visible-light camera, and captures an image of the subject person passing through the gate 10F and outputs the image to the controller 9. Through image analysis, the controller 9 identifies a target area TR in the space that the target part Ob of the subject person passes through, and outputs control signals for changing the angles of the light emission unit 2F and the photodetector 3F to angles corresponding to the target area TR.

Based on the control signals from the controller 9, the light emission unit 2F and the photodetector 3F actuate the movable units 5 (position changing units 52 and the angle changing units 53) to change the angle and focus of the light emission unit 2F and the photodetector 3F.

Here, the detecting unit 84F may capture a moving image of the subject person, a change in position of the target area TR may be identified by detecting the target area TR in each of a plurality of images through image analysis by the controller 9, and the light emission unit 2F and the photodetector 3F may change the angle and focus of the light emission unit 2F and the photodetector 3F by actuating the movable units 5 when the target area TR is in a predetermined position. According to such a structure, the angle and focus of the light emission unit 2F and the photodetector 3F can be accurately adapted to the position of the target area TR.

Alternatively, a change in position of the target area TR identified from a plurality of images through image analysis may be identified, a timepoint when the target area TR will be in a predetermined position may be estimated, and the angle and focus of the light emission unit 2F and the photodetector 3F may be changed by actuating the movable units 5 in accordance with the estimated timepoint. According to such a structure, the effect of a time lag caused by the operation time of the movable units 5 can be suppressed, and the angle and focus of the light emission unit 2F and the photodetector 3F can be more accurately adapted to the estimated position of the target area TR.

Alternatively, instead of actuating the movable units 5 to change the angle of the light emission unit 2F and the photodetector 3F and subsequently change the focus of the light emission unit 2F and the photodetector 3F, time-series measurement data may be recorded by emitting the laser beam L1 from the light emission unit 2F and receiving the signal light L2 by means of the photodetector 3F continuously for a predetermined amount of time during measurement, and measurement data corresponding to a specific timepoint may be selected from the recorded time-series data. Here, the specific timepoint may be the timepoint when the target area TR is present within a predetermined position range based on image analysis of a moving image captured by the detecting unit 84F.

Alternatively, in the focus adjustment, the distance to the target part Ob may be measured directly by emitting distance-measurement laser beams LM toward the target area TR by using the distance measuring units 83 installed inside the light emission unit 2F and the photodetector 3F, and the movable units 5 (position changing units 52) installed in the light emission unit 2F and the photodetector 3F may be actuated based on the measured distance to change the positions of the optical members 4. This makes it possible to perform the focus adjustment with higher accuracy.

Furthermore, the concentration of the substance in blood is measured by emitting the laser beam L1 from the light emission unit 2F to the subject portion Mp0 in the target part Ob of the subject person present in the specific target area TR in the space, and receiving the signal light L2 reflected from the subject portion Mp0 in the target part Ob by means of the photodetector 3F.

According to the device 1F having such a structure, the concentration of the substance in blood can be measured while identifying, with higher accuracy, the target part Ob of the subject person passing through an area within a predetermined larger area, such as the entrance to a building.

### (3) Measurement with respect to subject person passing through limited target area

In another embodiment of the device 1E, a target area TR in a space that the target part Ob of the subject person passes through may be limited to a specific area, and measurement may be performed only when the target part Ob of the subject person passes through the predetermined target area TR.

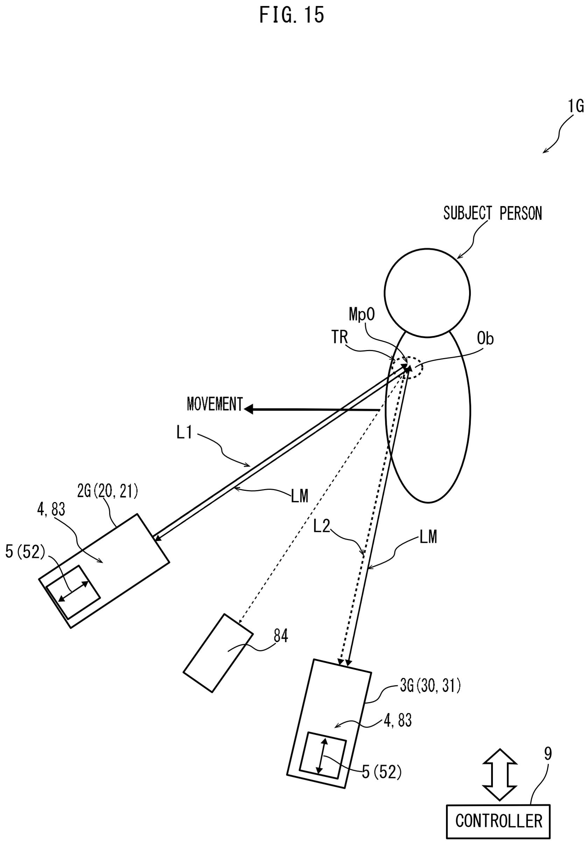

FIG. 15 is a schematic diagram illustrating a structure of a substance-in-blood concentration measurement device 1G according to a modification (also referred to hereinafter as "device 1G").

As illustrated in FIG. 15, the device 1G includes a light emission unit 2G and a photodetector 3G, optical members 4, position changing units 52, distance measuring units 83, a detecting unit 84, and a controller 9 that controls such elements.

The light emission unit 2G and the photodetector 3G respectively have the same structures as the light emission unit 2D and the photodetector 3D according to Embodiment 2. That is, the light emission unit 2G includes a housing 20 and a laser light source 21. Furthermore, the photodetector 3G includes a housing 30 and an infrared sensor 31.

Furthermore, in the device 1G, an optical member 4, the laser light source 21, and a position changing unit 52 are disposed in the housing 20, for example, similarly to the light emission unit 2D according to Embodiment 2. Furthermore, an optical member 4, the infrared sensor 31, and a position changing unit 52 are disposed in the housing 30, for example, similarly to the photodetector 3D according to Embodiment 2. Furthermore, in the device 1G, a distance measuring unit 83 that is the same as that in Embodiment 2 is disposed in each of the housings 20 and 30.

The detecting unit 84 is sensor for detecting whether or not the target part of the subject person is present within a limited specific target area TR in a space. Upon sensing the subject person within the target area TR, the detecting unit 84 outputs the result of the sensing to the controller 9.

The angles of the light emission unit 2G and the photodetector 3G are fixed to angles corresponding to the predefined target area TR. Furthermore, the light emission unit 2G and the photodetector 3G directly measure the distance to the target part Ob by emitting distance-measurement laser beams LM toward the target area TR using the distance measuring units 83 installed therein, and the movable units 5 (position changing units 52) in the light emission unit 2G and the photodetector 3G are actuated based on the measured distance to change the positions of the optical members 4. That is, when information indicating the distance to the target part Ob output from the distance measuring units 83 is within a predetermined range, the controller 9 actuates the movable units 5 (position changing units 52) and changes the positions of the optical members 4 immediately.

Furthermore, the concentration of the substance in blood is measured by emitting a laser beam L1 from the light emission unit 2G to the subject portion Mp0 in the target part Ob of the subject person present in the limited specific target area TR in the space, and receiving signal light L2 reflected from the subject portion Mp0 in the target part Ob by means of the photodetector 3G.

Alternatively, the positions of the optical members 4 may be set in advance by actuating the movable units 5 (position changing units 52) in a state in which the angles of the light emission unit 2G and the photodetector 3G are fixed to angles corresponding to the predetermined target area TR. In the device 1G, the optical members 4 are set to positions where the laser beam L1 can be emitted from the light emission unit 2G to the subject portion Mp0 in the target part Ob present within the predefined target area TR and the signal light L2 reflected from the subject portion Mp0 in the target part Ob present within the predefined target area TR can be received by means of the photodetector 3G.

Furthermore, during measurement, the concentration of the substance in blood is measured by emitting the laser beam L1 from the light emission unit 2G to the subject portion Mp0 in the target part Ob of the subject person and receiving the signal light L2 reflected from the subject portion Mp0 in the target part Ob by means of the photodetector 3G when the distance to the target part Ob is measured by the distance measuring unit 83 installed inside the photodetector 3G and distance information to the target part Ob that is output is within a predetermined range.

Alternatively, time-series measurement data may be recorded by emitting the laser beam L1 from the light emission unit 2G and receiving the signal light L2 by means of the photodetector 3G continuously for a predetermined amount of time during measurement in parallel with the operation of measuring the distance to the target part Ob using the distance measuring unit 83 installed inside the photodetector 3G. Thereafter, the concentration of the substance in blood may be measured by selecting, from the recorded time-series data of the signal light L2, measurement data of the signal light L2 corresponding to a timepoint when distance information to the subject portion Mp0 in the target part Ob output from the distance measuring unit 83 was within a predetermined range.

According to the device 1G having such a structure, the angle adjustment function can be omitted from the light emission unit 2G and the photodetector 3G, and the device can thus be simplified. Thus, the concentration of the substance in blood can be measured using a device that is lower in cost in a case such as that in which the target part Ob of subject persons will pass through a predetermined target area TR, such as when the device 1G is embedded in a wall of a narrow passage to measure subject persons passing by.

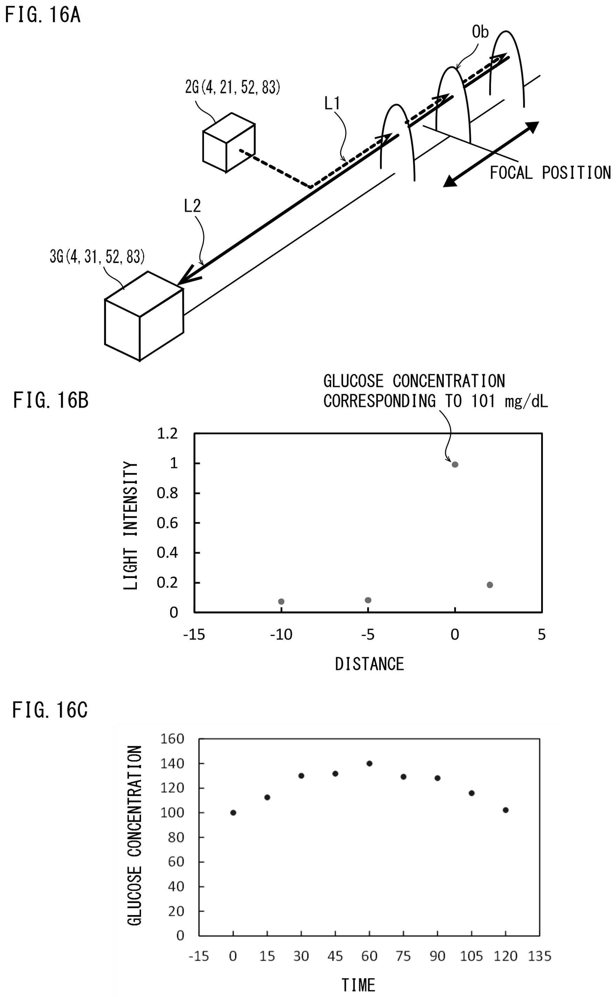

Next, using an example of the device 1G, the intensity of the signal light detected by the photodetector 3G was measured while changing the position of the target part Ob of a subject person relative to the photodetector 3G. FIG. 16A is a schematic diagram illustrating a structure of the example of the device 1G that was used for the measurement.

The measurement was performed according to the following procedure.
(i) The focal position was measured using a mid-infrared camera or the like equivalent to the photodetector 3G.
(ii) The photodetector 3G was installed at the same position as the detection surface of the mid-infrared camera.
(iii) The distance from the photodetector 3G to the focal position was measured using a distance measuring unit 83.
(iv) A mid-infrared laser beam was emitted from the light emission unit 2G while moving the target part Ob (finger) of a subject person gradually closer to the focal position from a distant position, and the light intensity of the signal light was measured. Furthermore, the distance from the target part Ob to the photodetector 3G during measurement was measured using a distance sensor.
(v) The relationship between the light intensity and the distance between the target part Ob and the photodetector 3G was obtained. Furthermore, the maximum value of light intensity was converted into a glucose concentration based on a mathematical formula identified in advance.

Note that, in the above, the incident angle of the laser beam relative to the surface of the skin of the target part Ob of the subject person was 0 degrees, and the angle of the optical axis passing through the center of the optical member 4 and the installation angle of the photodetector 3G relative to the surface of the skin of the target part Ob of the subject person were 0 degrees.

FIG. 16B is a diagram illustrating a relationship between positions of the target part Ob of the subject person measured using the example of the device 1G and the intensity of the signal light detected using the example of the device 1G, and FIG. 16C is a diagram illustrating a temporal change in the blood sugar level of the subject person. As illustrated in FIG. 16B, it was confirmed that the intensity of the signal light reaches the maximum when the target part Ob of the subject person is located at the focal position, and that an appropriate glucose concentration can be calculated from the maximum value of light intensity. Furthermore, results as illustrated in FIG. 16C were obtained by measuring the temporal change in blood sugar level in a state in which the target part Ob of the subject person is located at the focal position.

### (4) Measurement of distribution of concentration of substance in blood in subject person

In another embodiment of the device 1E, a distribution of the concentration of the substance in blood in the subject person can be measured.

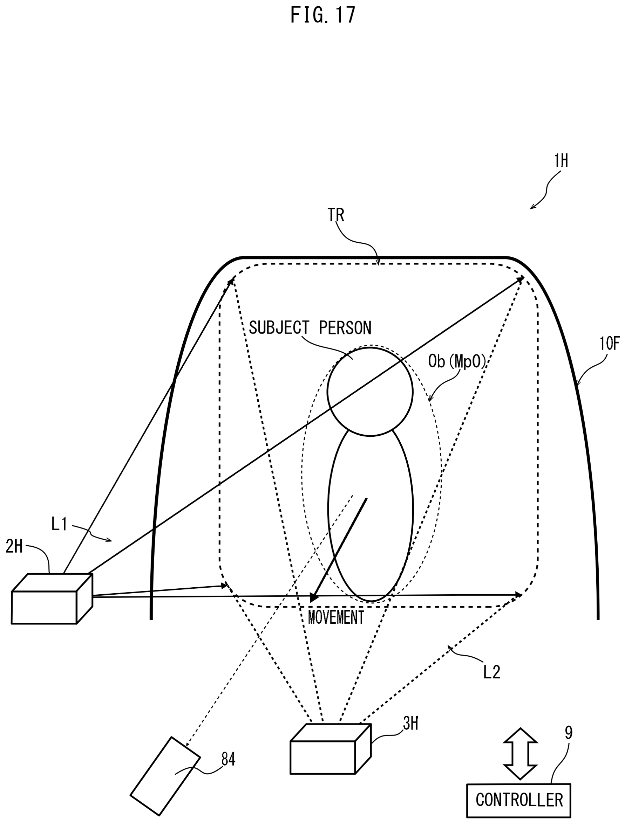

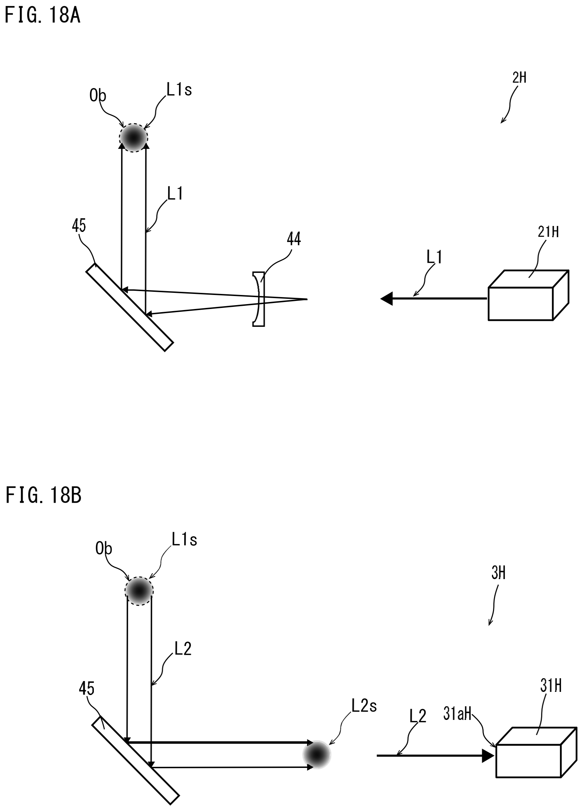

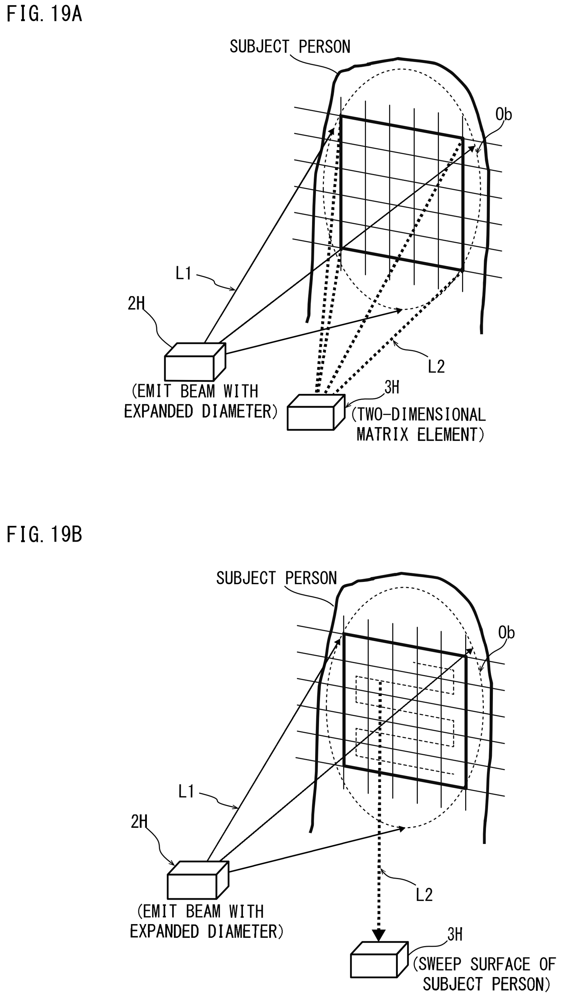

FIG. 17 is a schematic diagram illustrating a structure of a substance-in-blood concentration measurement device 1H according to a modification (also referred to hereinafter as "device 1H"), FIGS. 18A and 18B are schematic diagrams respectively illustrating structures of a light emission unit 2H and a photodetector 3H in the device 1H, and FIGS. 19A and 19B are diagrams for explaining operations by the substance-in-blood concentration measurement device 1H according to a modification.

As illustrated in FIG. 17, the device 1H includes a gate 10F that the subject person passes through, a light emission unit 2H and a photodetector 3H, a detecting unit 84, and a controller 9 that controls such elements.

As illustrated in FIG. 18A, the light emission unit 2H includes a laser light source 21H, a diffuser lens 44, and a mirror 45. The laser beam L1 emitted from the laser light source 21H is emitted after the beam diameter thereof is expanded by the diffuser lens 44 so that an image L1s of the laser beam covers the entirety of the target part Ob, which corresponds to the entire body of the subject person.

As illustrated in FIG. 18B, the photodetector 3H includes an infrared sensor 31H and a mirror 45. For example, the infrared sensor 31H is formed from a two-dimensional infrared sensor in which a plurality of elements are arranged in a matrix. The photodetector 3H includes the infrared sensor 31H having a light receiving surface 31aH corresponding to the size of an image L2s of the signal light L2 reflected from a laser beam irradiation region covering the entire target part Ob corresponding to the entire body of the subject person, and can measure a distribution of the intensity of the signal light L2 reflected from the target part Ob corresponding to the entire body of the subject person.

The detecting unit 84 is a detector that detects the subject person passing through the gate 10F. The detecting unit 84 detects whether or not the target part of the subject person is present within a specific target area TR set inside the gate 10F in a space. Upon sensing the subject person within the target area TR, the detecting unit 84 outputs the result of the sensing to the controller 9.

During measurement, the light emission unit 2H and the photodetector 3H may perform focus adjustment by performing transmission and reception for a predetermined amount of time as illustrated in FIG. 19A and acquiring three-dimensional intensity data of the signal light L2 over the predetermined amount of time. That is, the laser beam L1 is emitted from the light emission unit 2H for a predetermined amount of time, and three-dimensional intensity data of the signal light L2 over the predetermined amount of time is acquired by means of the plurality of elements arranged in a matrix in the infrared sensor 31H. Then, the time (distance) when the maximum intensity was obtained by each element may be extracted and set as the in-focus distance (acquired data) by selecting, from the obtained three-dimensional intensity data of the signal light L2 over the predetermined amount of time, intensity data indicating the maximum signal intensity in a time-direction signal sequence corresponding to each element. In this case, the light emission unit 2H and the photodetector 3H constitute a detecting unit that detects an area of presence of the target part Ob of the subject person in a space.

Alternatively, a plurality of visible-light cameras that capture images of the subject person passing through the gate 10F and output the images to the controller 9 are used as optional elements to create three-dimensional moving-image data of the subject person, and the distance from the light emission unit 2H and the photodetector 3H to the target part Ob is calculated. Then, the intensity data corresponding to the above-described distance in the signal sequence of each element may be selected from the three-dimensional intensity data of the signal light L2.

Alternatively, as illustrated in FIG. 19B, a distance measuring unit (unillustrated) that sweeps the body surface of the subject person passing through the gate 10F and measures the distance to each body surface portion may be used. In this case, the distance measuring unit measures distance data to each element portion obtained by breaking down the body surface of the subject person into a matrix and outputs the distance data to the controller 9, and the controller 9 calculates a matrix of distance data from the photodetector 3H to element portions of the body surface based on the obtained distance data. Then, based on the matrix of distance data, the controller 9 may select, for each element of the photodetector 3H, intensity data corresponding to the distance to the corresponding element portion of the body surface from the three-dimensional intensity data of the signal light L2 received by the elements of the photodetector 3H as a result of the laser beam L1 being emitted from the light emission unit 2H for a predetermined amount of time.

According to the device 1H having such a structure, a distribution of the concentration of the substance in blood can be measured using, as the subject portion Mp0 in the target part Ob, portions of the entire body of the subject person passing through the gate 10F that are exposed from the clothes worn by the subject person.

### (5) Another embodiment relating to measurement of distribution of concentration of substance in blood in subject person

Another embodiment in which a distribution of the concentration of the substance in blood in the subject person can be measured may be adopted.

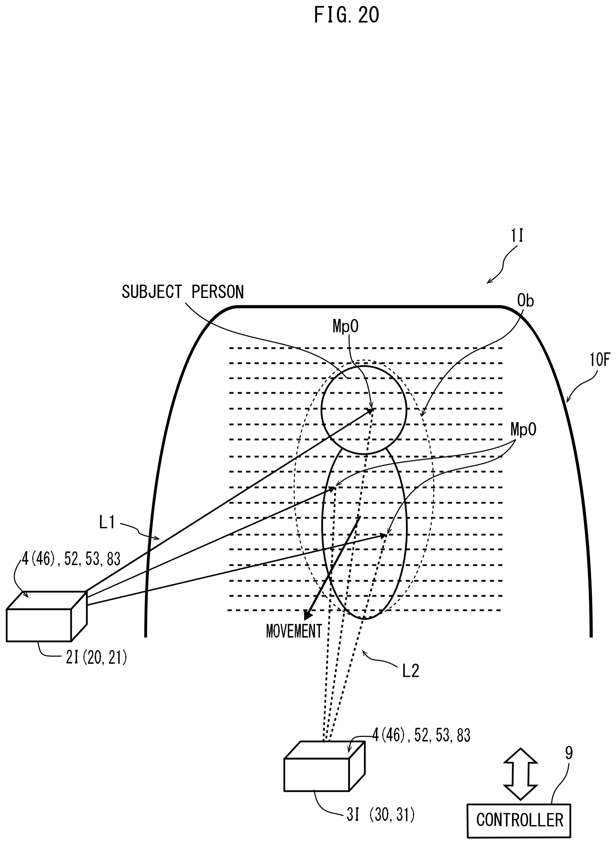

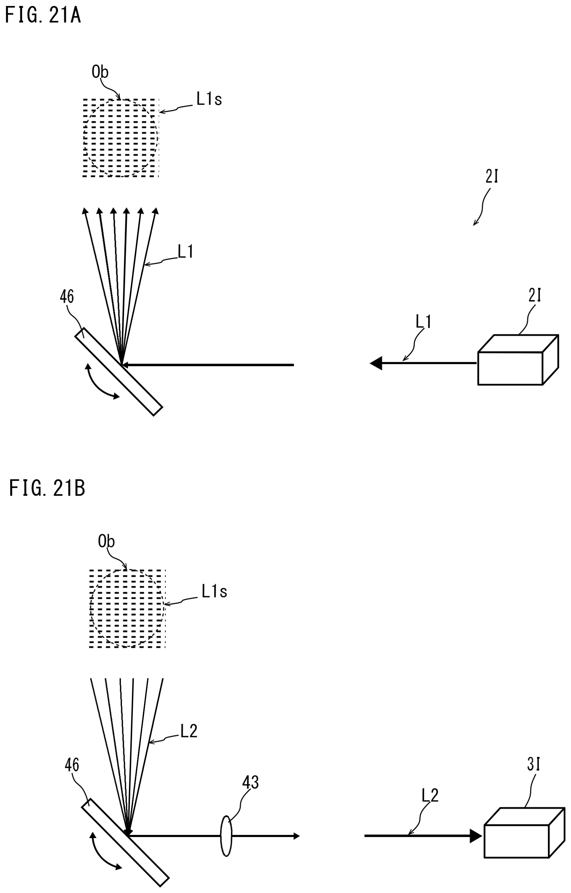

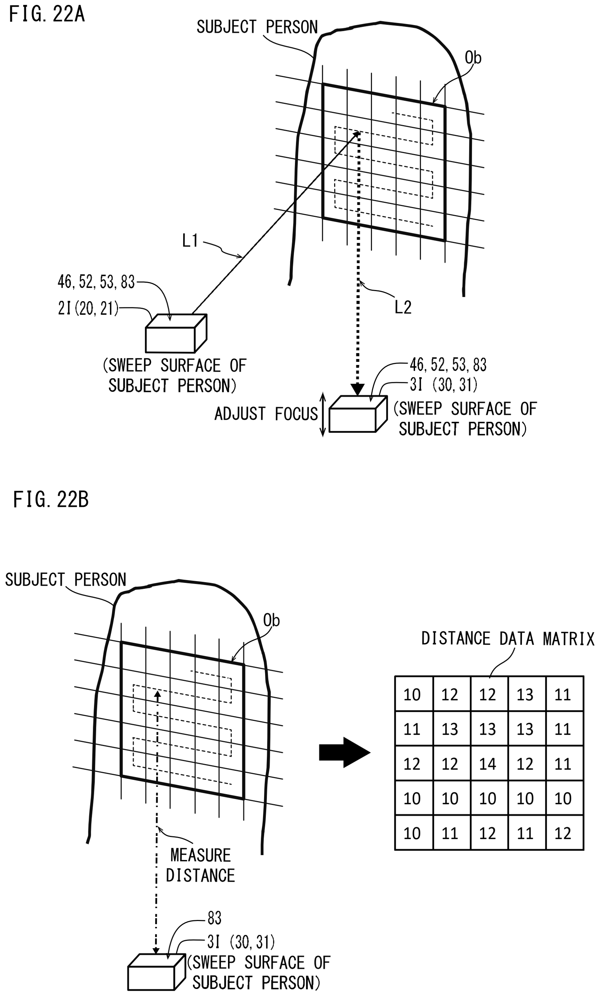

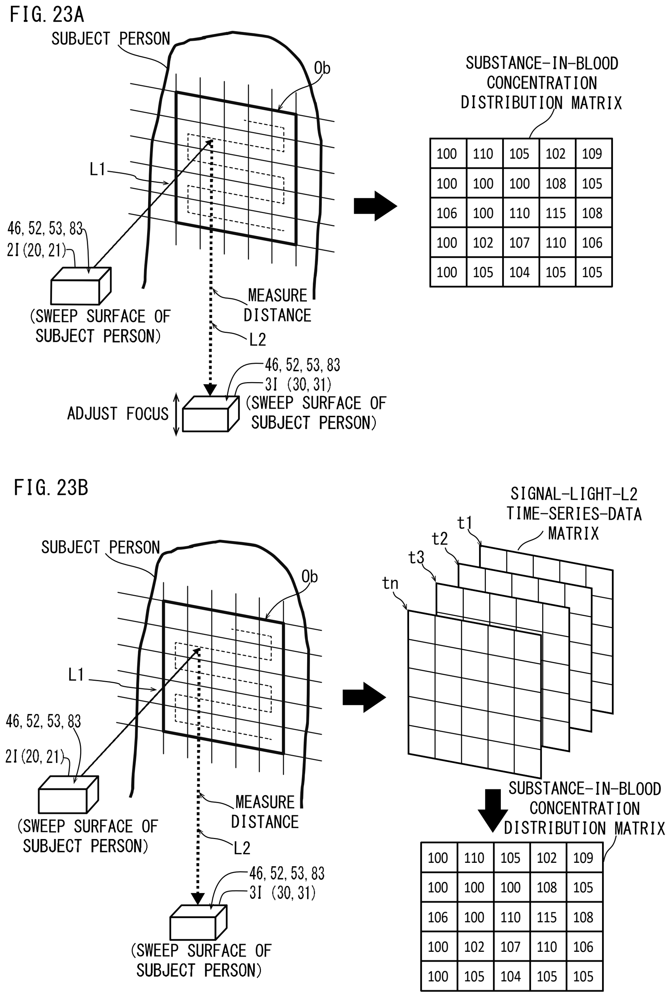

FIG. 20 is a schematic diagram illustrating a structure of a substance-in-blood concentration measurement device 1I according to a modification (also referred to hereinafter as "device 1I"), FIGS. 21A and 21B are schematic diagrams respectively illustrating structures of a light emission unit 2I and a photodetector 3I in the device 1I, and FIGS. 22A, 22B, 23A, and 23B are diagrams for explaining operations by the substance-in-blood concentration measurement device 1I according to a modification.

As illustrated in FIG. 20, the device 1I includes a gate 10F that the subject person passes through, a light emission unit 2I and a photodetector 3I, optical members 4, position changing units 52, angle changing units 53, distance measuring units 83, and a controller 9 that controls such elements.

As illustrated in FIG. 20, the light emission unit 2I includes a housing 20 and a laser light source 21. Furthermore, in the device 1I, the components of the light emission unit 2D according to Embodiment 2, i.e., an optical member 4, the laser light source 21, a position changing unit 52, and a distance measuring unit 83, are disposed in the housing 20, for example. Furthermore, in the device 1I, an angle changing unit 53 that is the same as that in Embodiment 2 is disposed in the housing 20. Furthermore, in the device 1I, a galvanometer scanner 46 is disposed in the housing 20. With the device 1I, as illustrated in FIG. 21A, a scan area L1s of the laser beam L1 emitted from a portion corresponding to the light emission unit 2I sweeps the entirety of the target part Ob, which corresponds to the entire body of the subject person, as a result of the emission direction of the laser beam L1 being changed by the galvanometer scanner 46.

As illustrated in FIG. 20, the photodetector 3I includes a housing 30 and an infrared sensor 31. Furthermore, in the device 1I, the components of the photodetector 3D according to Embodiment 2, i.e., an optical member 4, the infrared sensor 31, a position changing unit 52, and a distance measuring unit 83, are disposed in the housing 30, for example. Furthermore, in the device 1I, an angle changing unit 53 that is the same as that in Embodiment 2 is disposed in the housing 30. Furthermore, in the device 1I, a galvanometer scanner 46 is disposed in the housing 30. With the photodetector 3I, as illustrated in FIG. 21B, a distribution of the intensity of the signal light L2 reflected from the scan area L1s of the laser beam L1, which covers the entire target part Ob corresponding to the entire body of the subject person, can be measured as a result of the signal light L2, which is reflected from the target part Ob corresponding to the entire body of the subject person, being received in time series while the light-receiving direction is changed by the galvanometer scanner 46.

During operation of the light emission unit 2I and the photodetector 3I having such structures, the angle and focus of the light emission unit 2I and the photodetector 3I are changed by actuating the movable units 5 (position changing units 52 and angle changing units 53) as illustrated in FIG. 22A based on control signals from the controller 9. The angle and focus are changed by storing in advance, in the controller 9, optimal angles and optimal optical-member positions corresponding to scan positions of the laser beam L1 that are identified in advance, and changing the angles of the light emission unit 2I and the photodetector 3I to the angles corresponding to the scan positions of the laser beam L1 and transporting the optical members to the corresponding optimal positions.

Specifically, as illustrated in FIG. 22B, a distance measuring unit 83 sweeps the entire body of the subject person standing still inside the gate 10F to sequentially measure the distance to each element portion obtained by breaking down the body surface into a matrix and outputs the distance to the controller 9, and the controller 9 calculates a matrix of distance data from the photodetector 3I to element portions of the body surface based on the obtained distance data. Then, based on the distance data from the photodetector 3I to the individual element portions of the subject person, the controller 9 may identify the optimal angles and optimal optical-member positions corresponding to the scan positions of the laser beam L1 in advance and store the optimal angles and optimal optical-member positions in the controller 9.

Furthermore, in the focus adjustment, the light emission unit 2I and the photodetector 3I may, as illustrated in FIG. 23A, directly measure the distance to the target part Ob of the subject person passing through the gate 10F by emitting distance-measurement laser beams LM toward the target part Ob using the distance measuring units 83 installed therein, and the movable units 5 (position changing units 52) installed in the light emission unit 2I and the photodetector 3I may be actuated based on the measured distance to change the positions of the optical members 4. This makes it possible to perform the focus adjustment with higher accuracy. Furthermore, such a measurement method is more effective for focus adjustment performed in a case in which the subject person is moving, such as when the subject person is passing through the gate 10F. In this case, the distance measuring units 83 constitute detecting units that detect an area of presence of the target part Ob of the subject person in a space.

Furthermore, the concentration of the substance in blood is measured by emitting the laser beam L1 from the light emission unit 2I to the subject portion Mp0 in the target part Ob of the subject person present in a target area TR in the space, and receiving the signal light L2 reflected from the subject portion Mp0 in the target part Ob by means of the photodetector 3I.

Furthermore, in the device 1I, with respect to the individual element portions obtained by breaking down the body surface of the subject person into a matrix, time-series measurement data may be recorded by emitting the laser beam L1 from the light emission unit 2I and receiving the signal light L2 by means of the photodetector 3I continuously for a predetermined amount of time (t1 to tn), in parallel with the operation of measuring the distance to the target part Ob using the distance measuring unit 83 installed inside the photodetector 3I. Furthermore, the concentration of the substance in blood may be measured by selecting, from the obtained time-series data of the signal light L2, measurement data of the signal light L2 corresponding to distance information to the target part Ob, as illustrated in FIG. 23B. By performing such operations for individual element portions of the body surface of the subject person, a distribution of the concentration of the substance in blood on the body surface of the subject person can be measured.

Alternatively, similar to the device 1H, images of the subject person passing through the gate 10F may be captured using, as optional elements, visible-light cameras for creating three-dimensional moving-image data of the subject person, and the distance from the light emission unit 2I and the photodetector 3I to the target part Ob may be calculated based on the three-dimensional data of the subject person. Furthermore, the intensity data corresponding to the above-described distance in the signal sequence of each element may be selected from the time-series intensity data of the signal light L2 corresponding to the scan area L1.

According to the device 1I having such a structure, a distribution of the concentration of the substance in blood can be measured using, as the target part Ob, portions of the entire body of the subject person passing through the gate 10F that are exposed from the clothes worn by the subject person. Furthermore, in the device 1I, the diffusion of the laser beam L1 is suppressed compared to in the device 1H; due to this, the measurement device can be formed using a laser light source with lower output, and thus a distribution of the concentration of the substance in blood can be measured at lower cost.

Note that the distance to individual element portions of the body surface of a subject person passing through the gate 10F may be measured or calculated by capturing a moving image of the subject person passing through the gate 10F using a visible-light camera, and detecting the positions of individual element portions obtained by breaking down the body surface of the subject person into a matrix in each of a plurality of images through image analysis by the controller 9. The moving image may be captured using a single visible-light camera or a plurality of visible-light cameras provided at positions from which images of the subject person can be captured from different angles.

Alternatively, the distance to individual element portions of the body surface of the subject person passing through the gate 10F may be measured using an ultrasonic distance sensor in place of the distance measuring unit 83 illustrated in FIG. 22B.

### (6) Measurement with respect to limited target area position of which varies depending on subject person

In another embodiment of the device 1E, a goggle-type structure in which the forehead portion of a subject person is the target part Ob and which performs measurement while being worn on the face of the subject person may be adopted.

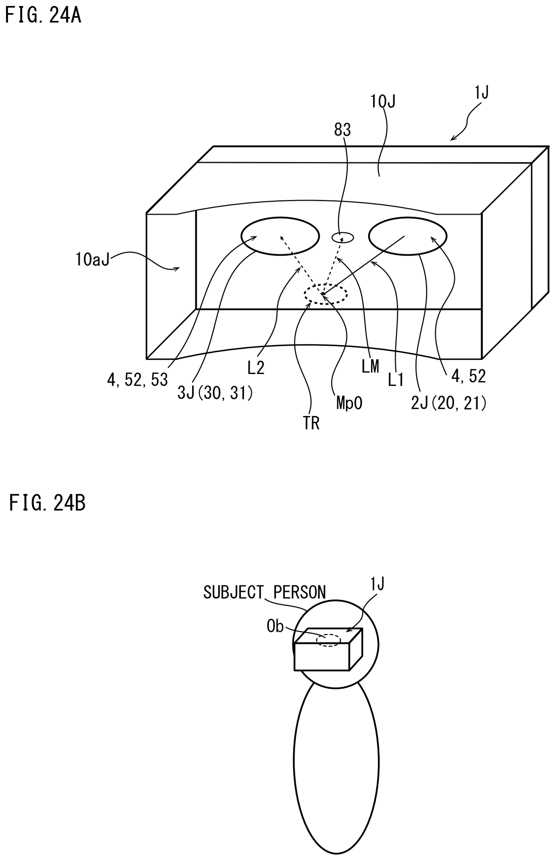

FIG. 24A is a schematic diagram illustrating a structure of a substance-in-blood concentration measurement device 1J according to a modification (also referred to hereinafter as "device J"), and FIG. 24B is a schematic diagram illustrating a state of the device 1J during measurement.

As illustrated in FIG. 24A, the device 1J includes goggles 10J, a light emission unit 2J and a photodetector 3J, optical members 4, position changing units 52, an angle changing unit 53, a distance measuring unit 83, and a controller (unillustrated) that controls such elements.

The light emission unit 2J and the photodetector 3J respectively have the same structures as the light emission unit 2D and the photodetector 3D according to Embodiment 2. That is, the light emission unit 2J includes a housing 20 and a laser light source 21. Furthermore, the photodetector 3J includes a housing 30 and an infrared sensor 31.

Furthermore, in the device 1J, an optical member 4, the laser light source 21, and a position changing unit 52 are disposed in the housing 20, for example, similarly to the light emission unit 2D according to Embodiment 2. Furthermore, an optical member 4, the infrared sensor 31, a position changing unit 52, and an angle changing unit 53 are disposed in the housing 30, for example, similarly to the photodetector 3D according to Embodiment 2.

The angle of the light emission unit 2J is fixed to an angle corresponding to a predefined target area TR. Specifically, the angle of the light emission unit 2J is set in advance so that, when the subject person attaches a rim portion of an opening 10aJ of the goggles 10J to the forehead portion of his/her face for measurement as illustrated in FIG. 24B, the direction of the laser beam L1 emitted from the light emission unit 2J is directed toward the direction of the forehead, which is the target part Ob of the subject person.

When the subject person attaches the goggles 10J to the forehead portion, the distance measuring unit 83 measures the distance to the forehead, which is the target part Ob, by emitting a distance-measurement laser beam LM toward the forehead, which is the target part Ob, and outputs the result of the measurement to the controller, and the controller corrects the target area TR based on the measured distance. In this case, the distance measuring unit 83 constitutes a detecting unit that detects an area of presence (target area TR) of the target part Ob of the subject person in a space.

Based on the target area TR corrected based on the measured distance, the light emission unit 2J adjusts the focus to the target area TR by actuating the movable unit 5 (position changing unit 52) of the light emission unit 2J to change the position of the optical member 4.

On the other hand, in the photodetector 3J, the movable unit 5 (position changing unit 52 and angle changing unit 53) of the photodetector 3J is actuated based on the target area TR corrected based on the measured distance to change the position of the optical member 4 and the angle of the photodetector 3J so as to be adjusted to the target area TR. Thus, the light emission unit 2J and the photodetector 3J are set in a state such that the region to which the laser beam L1 is condensed is capable of matching the specific region in the subject portion Mp0 in the forehead portion, which is the target part Ob, and a focused image of signal light L2 from a region to which the laser beam is condensed can be formed on the photodetector 3J.

Furthermore, the concentration of the substance in blood is measured by emitting the laser beam L1 from the light emission unit 2J to the forehead, which is the target part Ob of the subject person, and receiving the signal light L2 reflected from the target part Ob by means of the photodetector 3J.

According to the device 1J having such a structure, the concentration of the substance in blood in the target part Ob, the position of which varies depending on the subject person, can be measured simply and accurately by the subject person simply attaching the goggles 10J to the forehead portion.

### <Section Summary>

(1) As described above, in substance-in-blood concentration measurement devices 1E, 1F, and 1J according to Embodiment 3,which measures a concentration of a substance in blood included in the blood in a subject portion Mp0 in a target part Ob of a subject person, the substance-in-blood concentration measurement device 1E, 1F, and 1J includes: a measuring unit (83, 84) that measures information relating to the target part Ob of the subject person; a light emission unit 2 (2E, 2F, 2J) that emits a laser beam L1 to a specific region in the subject portion in the target part Ob; a photodetector 3 (3E, 3F, 3J) that receives signal light L2 reflected from the subject person based on the laser beam L1 and detects an intensity of the signal light L2; an optical member 4 that condenses the laser beam L1 to the specific region in the subject portion Mp0 in the target part Ob, and forms, on the photodetector 3 (3E, 3F, 3J), a focused image of the signal light L2 from a region to which the laser beam L1 is condensed in the subject portion Mp0; a movable unit 5 (52, 53) that changes a state of the optical member 4; and a controller 9 that, based on the intensity of the signal light L2, obtains a concentration of the substance in blood in the region to which the laser beam L1 is condensed. The movable unit 5 (52, 53) changes the state of the optical member 4 based on the information relating to the target part Ob.

That is, the substance-in-blood concentration measurement devices 1E, 1F, and 1J according to Embodiment 3 are characterized in that the measuring unit includes a detecting unit (83, 84) that detects, as the information relating to the target part Ob, an area of presence (target area TR) of the target part Ob of the subject person in a space. The movable unit (52, 53) changes a position and an angle of the optical member with respect to each of a plurality of positions in the space adaptively so that the region to which the laser beam L1 is condensed is capable of matching the specific region in the subject portion Mp0 and a focused image of the signal light L2 from the region to which the laser beam L1 is condensed can be formed on the photodetector 3. Based on the area of presence (target area TR) of the target part Ob detected by the detecting unit (83, 84), the movable unit selects a measurement-target position from among the plurality of positions and changes the position and the angle of the optical member 4 so as to be adapted to the measurement-target position, whereafter the light emission unit 2 emits the laser beam L1, the photodetector 3 receives the signal light L2 based on the laser beam L1 and detects the intensity of the signal light L2, and the controller 9 measures the concentration of the substance in blood based on the intensity of the signal light L2.

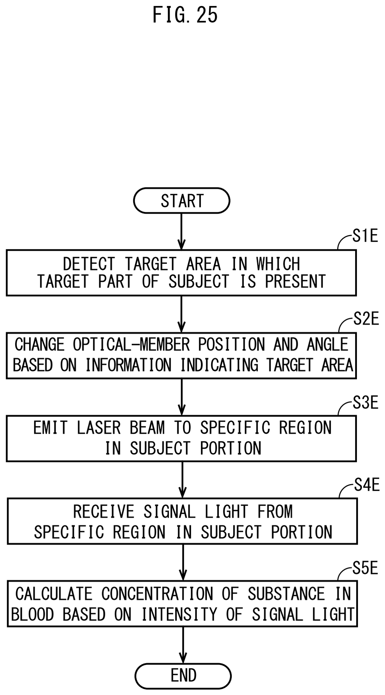

In the devices 1E, 1F, and 1J having the above-described structure, the controller 9 performs an operation for measuring the concentration of the substance in blood in the blood in the subject portion Mp0 in the target part Ob of the subject person through the following processing. FIG. 25 is a flowchart illustrating a substance-in-blood measurement operation by the devices 1E, 1F, and 1J.

First, as illustrated in FIG. 25, as the information relating to the target part Ob of the subject person, the area of presence (target area TR) of the target part Ob of the subject person in the space is detected by the measuring units constituted from the detecting units (83, 84) (step S1E).

Next, based on the information indicating the area of presence of the target part Ob, the state of the optical members 4 are changed by the optical-member movable units (52, 53) constituting the movable units 5 (step S2E). Specifically, the optical-member movable units 52 change the positions of the optical members 4 so that the region to which the laser beam L1 is condensed is capable of matching the specific region in the subject portion Mp0, and a focus image of the signal light L2 from the region to which the laser beam L1 is condensed can be formed on the photodetector 3. Furthermore, a measurement-target position is selected from among a plurality of positions based on the area of presence (target area TR) of the target part Ob detected by the detecting units (83, 84), and the optical-member movable units 53 change the angles of the optical members 4 so as to be adapted to the measurement-target position.

Next, the laser beam L1 is emitted to the specific region in the subject portion Mp0 in the target part Ob by the light emission unit 2E, 2F, 2J (step S3E), and the signal light L2 from the subject portion Mp0 based on the laser beam L1 is received and the intensity of the signal light L2 is detected by the photodetector 3E, 3F, 3J (step S4E). Here, the laser beam L1 is condensed to the specific region in the subject portion Mp0 in the target part Ob by the optical members 4, and a focused image of the signal light L2 from the region to which the laser beam L1 is condensed in the subject portion Mp0 is formed on the photodetector by the optical members 4.

Next, the concentration of the substance in blood in the region to which the laser beam L1 is condensed is obtained based on the intensity of the signal light L2 (step S5E), and the processing is terminated.

According to the devices 1E and 1F having such a structure, the concentration of the substance in blood can be measured while identifying, with higher accuracy, the target part Ob of the subject person when the subject person is passing through a predetermined path, such as a passage inside a building or an athletic track, or when the subject person is passing through a predetermined area, such as an entrance to a building.

Thus, the devices 1E and 1F are widely applicable to various scenes in human life, such as support and services relating to healthcare, wellness, eating and drinking, sports, education, entertainment, etc.

Furthermore, according to the device 1J, the concentration of the substance in blood can be measured simply and accurately by the subject person simply attaching the goggles 10J to the forehead portion.

(2) Substance-in-blood concentration measurement devices 1G and 1H according to Embodiment 3 are characterized by including: a detecting unit 84 that detects whether or not the target part Ob of the subject person is present within a specific area (target area TR) in a space; a light emission unit 2 (2G, 2H) that emits a laser beam L1 to a specific region in the subject portion Mp0 in the target part Ob; a photodetector 3 (3G, 3H) that receives signal light L2 from the subject person based on the laser beam L1 and detects an intensity of the signal light L2; an optical member 4 that condenses the laser beam L1 to the specific region in the subject portion Mp0 in the target part Ob located in the specific area, and forms, on the photodetector 3, a focused image of the signal light L2 from a region to which the laser beam L1 is condensed in the subject portion Mp0; and a controller 9 that, based on the intensity of the signal light L2, obtains a concentration of the substance in blood in the region to which the laser beam is condensed L1. When the detecting unit 84 detects that the target part Ob is present within the specific area (target area TR) in the space, the light emission unit 2 (2G, 2H) emits the laser beam L1, the photodetector 3 (3G, 3H) receives the signal light L2 based on the laser beam L1 and detects the intensity of the signal light L2, and the controller 9 measures the concentration of the substance in blood based on the intensity of the signal light L2.

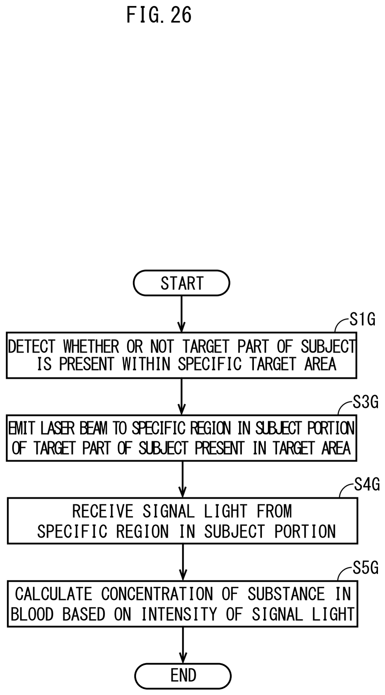

In the devices 1G and 1H having the above-described structure, the controller 9 performs an operation for measuring the concentration of the substance in blood in the blood in the subject portion Mp0 in the target part Ob of the subject person through the following processing. FIG. 26 is a flowchart illustrating a substance-in-blood measurement operation by the devices 1G and 1H.

First, as illustrated in FIG. 26, as the information relating to the target part Ob of the subject person, it is detected by the detecting unit 84 whether or not the target part Ob of the subject person is present within a specific area (target area TR) in the space (step S1G).

Next, with respect to the target part Ob of the subject person present in the limited specific target area TR in the space, the laser beam L1 is emitted to the specific region in the subject portion Mp0 in the target part Ob by the light emission unit 2G (step S3G), and the signal light from the subject person based on the laser beam L1 is received and the intensity of the signal light L2 is detected by the photodetector 3G (step S4G).

Here, the angles of the light emission unit 2G and the photodetector 3G are set so as to be fixed to angles corresponding to the predefined target area TR. Furthermore, the position of the optical member 4 is set to a position where the laser beam L1 can be emitted to the target part Ob present in the target area TR and the reflected signal light L2 can be received.

Furthermore, the distance to the target part Ob may be measured using a distance measuring unit (unillustrated), and the laser beam L1 may be emitted from the light emission unit 2G to the target part Ob of the subject person when the distance information to the target part Ob that is output is within a predetermined range. Alternatively, time-series measurement data may be recorded by emitting the laser beam L1 and receiving the signal light L2 continuously for a predetermined amount of time, and measurement data of the signal light L2 corresponding to a timepoint when the distance information to the target part Ob output from the distance measuring unit 83 was within the predetermined range may be selected.

Here, the laser beam L1 can be condensed to the specific region in the subject portion Mp0 in the target part Ob by the optical members 4, and a focused image of the signal light L2 from the region to which the laser beam L1 is condensed in the subject portion Mp0 can be formed on the photodetector by the optical members 4.

Alternatively, in steps S3G and S4G, an image L1s of the laser beam may be emitted by the light emission unit 2H in a state such that the beam diameter thereof is expanded by a diffuser lens 44 so that the image L1s covers the entirety of the target part Ob, which corresponds to the entire body of the subject person. Furthermore, by means of a two-dimensional infrared sensor (photodetector 3H) that has a light receiving surface 31aH corresponding to the size of an image L2s of the signal light L2 reflected from a laser beam irradiation region corresponding to the entire body of the subject person, a distribution of the intensity of the signal light L2 reflected from the target part Ob corresponding to the entire body of the subject person may be measured.

Next, the concentration of the substance in blood in the region to which the laser beam L1 is condensed is obtained based on the intensity of the signal light L2 (step S5G), and the processing is terminated.

According to such a structure, the angle adjustment function can be omitted from the light emission unit 2G and the photodetector 3G, and the device can thus be simplified. Thus, the concentration of the substance in blood can be measured using a device that is lower in cost in a case such as that in which it is known that the target part Ob of subject persons will pass through a predetermined target area TR.

Furthermore, the use of the diffuser lens 44 and the two-dimensional infrared sensor for the light emission unit 2G and the photodetector 3G, respectively, makes it possible to measure a distribution of the concentration of the substance in blood using, as the target part Ob, portions of the entire body of the subject person passing through the gate 10F that are exposed from the clothes worn by the subject person.

(3) A substance-in-blood concentration measurement device 1I according to Embodiment 3 is characterized by including, as the measuring unit measuring information relating to the target part Ob of the subject person, a distance measuring unit 83 that detects, as the information relating to the target part Ob, an area of presence of the target part Ob of the subject person in a space. The movable unit 5 (52, 53) can change a position and an angle of the optical member 4 with respect to each of a plurality of positions in the space adaptively so that the region to which the laser beam L1 is condensed is capable of matching the specific region in the subject portion Mp0 in the target part Ob and a focused image of the signal light L2 from the region to which the laser beam L1 is condensed can be formed on the photodetector 3 (3I). The detecting unit 83 detects the area of presence of the target part Ob of the subject person, the movable unit 5 (52, 53) changes the position and the angle of the optical member 4 so as to be adapted to each of the plurality of positions, to each of the plurality of positions, the light emission unit 2 (2I) emits the laser beam L1, from each of the plurality of positions, the photodetector 3 receives the signal light L2 based on the laser beam L1 and detects the intensity of the signal light L2, and the controller 9 measures the concentration of the substance in blood based on the intensity of the obtained signal light L2, and further selects a measurement-target position from among the plurality of positions based on the area of presence of the target part Ob and selects the concentration of the substance in blood corresponding to the measurement-target position.

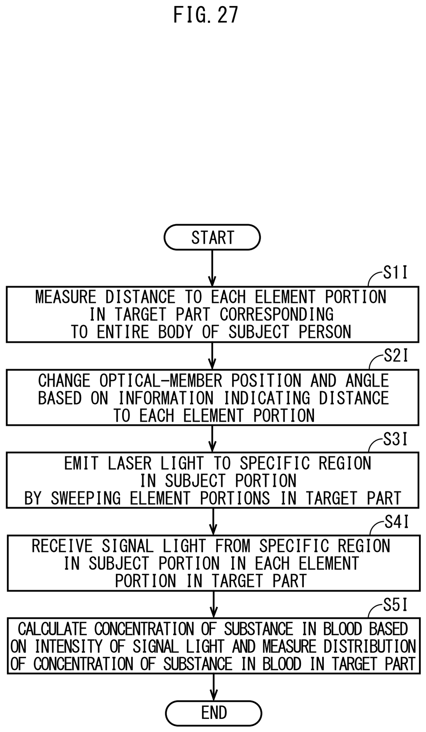

In the device 1I having the above-described structure, the controller 9 performs an operation for measuring the concentration of the substance in blood in the blood in the subject portion Mp0 in the target part Ob of the subject person through the following processing. FIG. 27 is a flowchart illustrating a substance-in-blood measurement operation by the device 1I.

First, as illustrated in FIG. 27, as the information relating to the target part Ob of the subject person, the distance to the target part Ob is measured by the measuring units constituted from the distance measuring units 83 (step S1I). That is, with the device 1I, the area of presence of the target part Ob of the subject person in the space is detected by measuring the distance to the target part Ob using the distance measuring units 83. Specifically, the distance measuring units 83 sweeps the entire body of the subject person standing still inside the gate 10F to sequentially measure the distance to each element portion obtained by breaking down the body surface into a matrix.

Next, based on the information relating to the target part Ob, the state of the optical members 4 is changed by the optical-member movable units (52, 53) constituting the movable units 5 (step S2I). Specifically, the optical-member movable units 53 change the angle of the optical members 4 so as to be adapted to measurement-target positions of the distance measuring units 83. Furthermore, based on the distance to the target part Ob detected by the distance measuring units 83, the optical-member movable units 52 change the position of the optical members 4 with respect to each of the plurality of positions in the space so that the region to which the laser beam L1 is condensed is capable of matching the specific region in the subject portion Mp0, and a focused image of the signal light L2 from the region to which the laser beam L1 is condensed can be formed on the photodetector 3.

Next, the laser beam L1 is emitted to the specific region in the subject portion Mp0 in the target part Ob corresponding to the entire body of the subject person by the light emission unit 2I (step S3I), and the signal light L2 from the target part Ob corresponding to the entire body of the subject person based on the laser beam L1 is received and the intensity of the signal light L2 is detected by the photodetector 3I (step S4I).

Specifically, with the device 1I, the laser beam L1 is emitted such that a scan area L1s of the laser beam L1 sweeps the entirety of the target part Ob corresponding to the entire body of the subject person as a result of the emission direction of the laser beam L1 emitted from the light emission unit 2I being sequentially changed by the galvanometer scanner 46. Furthermore, with the photodetector 3I, a distribution of the intensity of the signal light L2 reflected from the scan area L1s of the laser beam L1, which covers the entire target part Ob corresponding to the entire body of the subject person, is measured as a result of the signal light L2 reflected from the target part Ob corresponding to the entire body of the subject person being received in time series while the light-receiving direction is changed by the galvanometer scanner 46.

Here, the laser beam L1 is condensed to the specific region in the subject portion Mp0 in the target part Ob by the optical members 4, and a focused image of the signal light L2 from the region to which the laser beam L1 is condensed in the subject portion Mp0 is formed on the photodetector by the optical members 4.

Next, the concentration of the substance in blood in the region to which the laser beam L1 is condensed is obtained based on the intensity of the signal light L2 (step S5I), and the processing is terminated.

According to the device 1I having such a structure, a distribution of the concentration of the substance in blood can be measured using, as the target part Ob, portions of the entire body of the subject person passing through the gate 10F that are exposed from the clothes worn by the subject person.

### «Evaluation Test»

In the following, results obtained by evaluating performance through an evaluation test using an example of the device 1 and a comparative example will be described.

### (Example)

A prototype usable as an example of the device 1 was produced and evaluated.

In the example of the device 1, a laser light source having a wavelength selected from the range of 2.5-12 µm, inclusive, was used as the laser light source 21 of the light emission unit 2. A near-infrared/mid-infrared sensor was used as the photodetector 3.

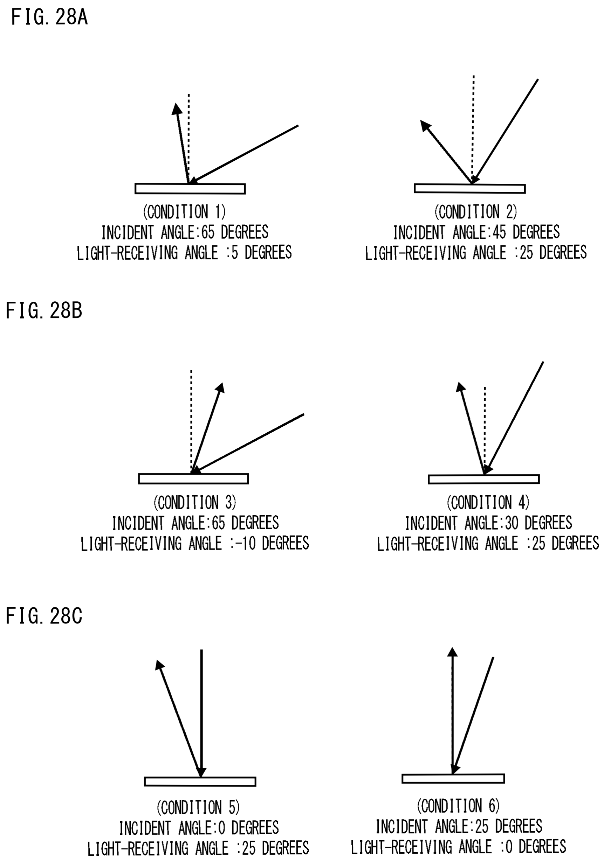

FIGS. 28A to 28C are diagrams illustrating combination conditions of the incident angle of the laser beam L1 relative to the surface of the skin of the target part Ob and the light-receiving angle (installation angle) of the photodetector 3 relative to the surface of the skin of the target part Ob in the evaluation test. As illustrated in FIGS. 28A to 28C, the combinations of the incident angle and the light-receiving angle (installation angle) were: (condition 1) incident angle: 65 degrees, light-receiving angle: 5 degrees; (condition 2) incident angle: 45 degrees, light-receiving angle: 25 degrees; (condition 3) incident angle: 65 degrees, light-receiving angle: -10 degrees; (condition 4) incident angle: 30 degrees, light-receiving angle: 25 degrees; (condition 5) incident angle: 0 degrees, light-receiving angle: 25 degrees; and (condition 6) incident angle: 25 degrees, light-receiving angle: 0 degrees.

As the condenser lens 41 of the optical member 4, a lens having a focal length of f = 25 mm was used. The condenser lens 41 was installed at a position that is approximately 50 mm from both the laser-beam condensing region in the subject portion Mp0 and the light receiving surface 31a of the photodetector 3, and the photodetector 3 was installed at the image transfer (focus) position from the laser-beam condensing region. Here, the focus depth of the detector was set to a depth selected from the range of 0.5-3.5 mm, inclusive, inwards of the surface of the skin of the living body Ob, and was set to 1.5 mm in the present example.

### (Comparative Example)

Flash Glucose Monitoring (FGM)-based glucose concentration measurement results measured using a commercially available glucose measurement device as a comparative example were used.

### (Test method)

Using the back side of the index finger of an adult male subject person as the subject, glucose concentration measurement was performed at substantially fixed time intervals using the example based on the device 1 and the FGM-based comparative example. Measurement using the device 1 was performed using the living body Ob, and the measurement using the example and the measurement using the comparative example were performed at the same time.

### (Test results)

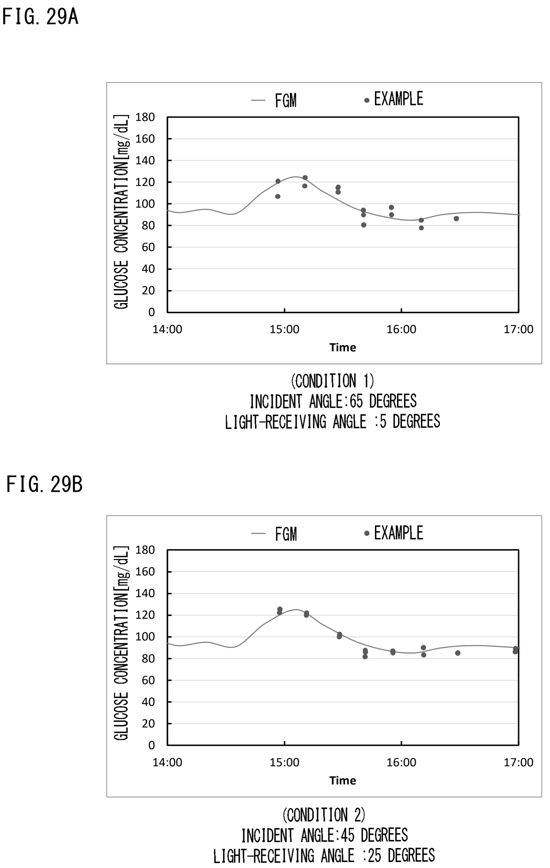

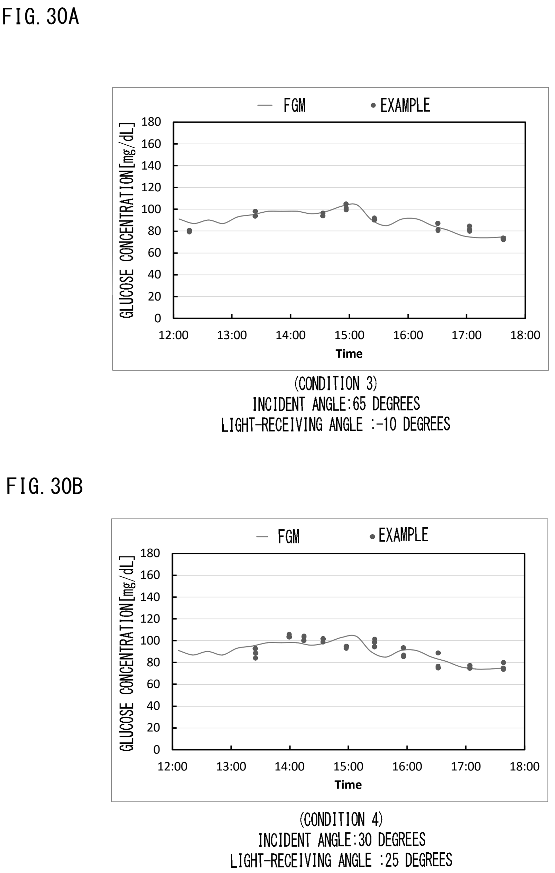

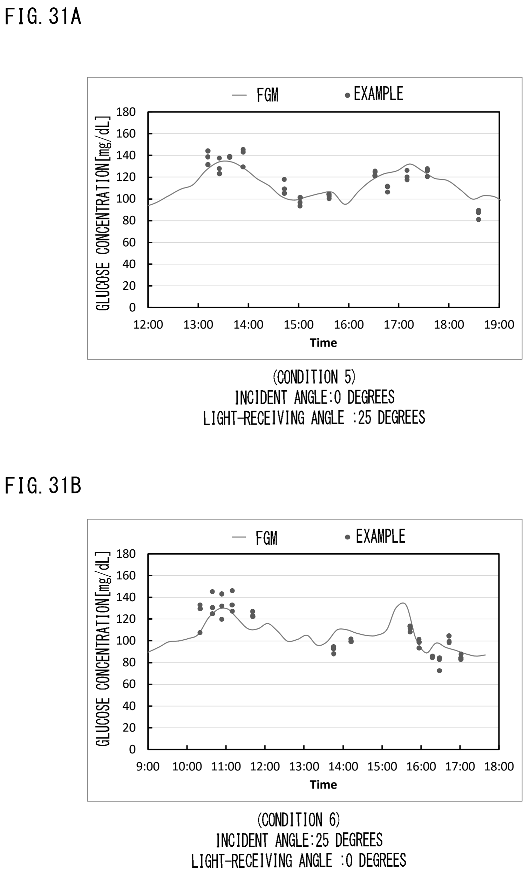

FIGS. 29, 30, and 31 respectively illustrate, for conditions 1 and 2, conditions 3 and 4, and conditions 5 and 6, results of the substance-in-blood concentration measurement test in which the example of the device 1 and the FGM-based comparative example were used. As illustrated in FIGS. 29, 30, and 31, for any of the combinations of incident angle and light-receiving angle in conditions 1 to 6, a measurement value obtained using the example was within a range of ±5%, in average, from a measurement value obtained using the comparative example at the same timepoint, and a high level of consistency could be seen between the results obtained using the example and the results obtained using the comparative example, which is a commercially available product indicating the present state of the art. Thus, it was confirmed that, according to the device 1, accurate measurement can be stably performed regardless of the combination of an incident angle and a light-receiving angle, within the incident angle range of 0 degrees to 65 degrees, inclusive, and the light-receiving angle range of -10 degrees to 25 degrees, inclusive. However, without limitation to these angular ranges, the incident angle can be selected from within the range of 0 degrees to 80 degrees, inclusive, and the light-receiving angle can be selected from within the range of -80 degrees to 80 degrees, inclusive.

### « Conclusion»

In conventional noninvasive substance-in-blood concentration measurement devices, measurement is performed using a finger as the measurement-target part in a state in which the palm side of the finger is placed in contact with a light-emission window provided on the outer surface of the measurement device.

Thus, there is a problem that it is difficult to measure concentrations of substances in blood in various scenes in daily life and from subject persons who are moving actively because the subject person needs to be in contact with a part of the measurement device even though the measurement is optical.

In contrast, according to the substance-in-blood concentration measurement device, the substance-in-blood concentration measurement method, and the program according to aspects of the present disclosure, an accurate measurement of the concentration of a substance in blood can be performed more simply and stably by taking advantage of the characteristics of optical measurement and using, as the measurement-target part, parts of the body of a subject person where the skin is exposed, which include not only the fingers but also other parts.

Thus, the present disclosure makes it possible to widely apply laser-beam-based measurement of the concentration of substances in blood to various scenes in human life, such as support and services relating to healthcare, wellness, eating and drinking, sports, education, entertainment, etc., and can realize a substance-in-blood concentration measurement device, a substance-in-blood concentration measurement method, and a program that contribute to improving the quality of life.

### <<Other Modifications>>

Up to this point, specific structures of the present disclosure have been described taking embodiments as examples, but the present disclosure is not limited by the above embodiments in any way, except for essential characteristic constituent elements thereof. For example, embodiments that can be obtained by applying various modifications to the embodiments, and embodiments that can be realized by combining constituent elements and functions in the embodiments within the scope of the present invention are also included in the present disclosure.

(1) In the above-described embodiments, embodiments have been described taking glucose as an example of the measurement-target substance to be detected by the substance-in-blood concentration measurement device. However, components in blood that can be detected by the substance-in-blood concentration measurement device according to the present disclosure are not limited to the above, and the device can be widely used for other detection targets by changing the wavelength of the laser beam L1 emitted by the light emission unit 2 according to the type of component in blood.
(2) In the above-described embodiments, embodiments of the substance-in-blood concentration measurement device have been described taking as an example an optical system formed from an optical member 4 that includes a condenser lens 41 between the subject portion Mp0 and the photodetector 3. However, as long as the substance-in-blood concentration measurement device according to the present disclosure forms, on the photodetector 3, a focused image from the signal light L2 reflected from the blood-vessel region in the target part Ob of the subject person, the structure of the light-receiving-side optical system may be changed, as appropriate. For example, a structure in which a plurality of lenses are used or a structure in which a mirror is disposed in the middle of the optical path may be adopted.
(3) The orders in which steps are executed in the embodiments are examples for specifically describing the present invention, and orders other than the orders above may be adopted. Furthermore, some of the steps may be executed concurrently with (in parallel with) other steps.

Furthermore, at least some of the functions in the embodiments and the modifications thereof may be combined with one another.

### « Supplement»

The embodiments described above are preferred specific examples of the present invention. Numerical values, shapes, materials, constituent elements, arrangements, positions, and connections of constituent elements, processes, order of processes, and the like described above are illustrative examples of embodiments, and are not intended to limit the present invention. Furthermore, among the constituent elements in the embodiments, those that are not recited in the independent claims, which represent the highest-level concepts of the present invention, are described as optional constituent elements constituting preferred embodiments.

Furthermore, the order according to which the above-described method is executed is an example for specifically describing the present invention, and an order other than the order above may be adopted. Furthermore, parts of the above-described method may be performed concurrently with (in parallel with) other methods.

Furthermore, in order to facilitate understanding of the invention, constituent elements in the drawings mentioned in the embodiments are not necessarily drawn to scale. Furthermore, the present invention is not limited by the description of the above embodiments, and can be modified, as appropriate, within the scope of the present invention.

Furthermore, all numbers used above are examples for specifically describing the present invention, and the present invention is not limited by the numbers used as examples.

### [Industrial Applicability]

The substance-in-blood concentration measurement device, substance-in-blood concentration measurement method, and program according to aspects of the present disclosure can be used in medical devices for the daily measurement of states of substances in blood, such as the blood sugar level and blood lipid level, in the prevention and treatment of lifestyle-related diseases. Furthermore, the substance-in-blood concentration measurement device, substance-in-blood concentration measurement method, and program according to aspects of the present disclosure are widely applicable to services for supporting eating and drinking, healthcare, sports, alcohol control, and security inspection for drugs, etc.

### [Reference Signs List]

- 1, 1A, 1B, 1C, 1D: Substance-in-blood concentration measurement device
- 10F: Gate
- 10J: Goggles
- 2: Light emission unit
21 Laser light source
22 Diaphragm
23 Condenser lens
- 3: Photodetector
31 Infrared sensor
32 Beam splitter
33 Dichroic mirror
34 Visible-light camera
- 4: Optical member
41 Condenser lens
42 Optical fiber
43 Condenser lens
44 Diffuser lens
45 Mirror
46 Galvanometer scanner
- 5: Optical-member movable unit
51 Optical-member holder
52 Position changing unit (linear transportation mechanism)
53 Angle changing unit
- 6: Visible laser light source (position measuring unit)
- 8: Main-body portion
81 Display unit
82 Camera (position measurement device)
83 Distance measuring unit (distance measurement device)
- 9: Controller

## Claims

1. A substance-in-blood concentration measurement device that measures a concentration of a substance in blood included in the blood in a subject portion in a target part of a subject person, the substance-in-blood concentration measurement device comprising:
a measuring unit that measures information relating to the target part of the subject person;
a light emission unit that emits a laser beam to a specific region in the subject portion in the target part;
a photodetector that receives signal light reflected from the subject person based on the laser beam and detects an intensity of the signal light;
an optical member that condenses the laser beam to the specific region in the subject portion in the target part, and forms, on the photodetector, a focused image of the signal light from a region to which the laser beam is condensed in the subject portion;
an optical-member movable unit that changes a state of the optical member; and
a controller that, based on the intensity of the signal light, obtains a concentration of the substance in blood in the region to which the laser beam is condensed.

2. The substance-in-blood concentration measurement device according to claim 1,
wherein the optical-member movable unit changes the state of the optical member based on the information relating to the target part.

3. The substance-in-blood concentration measurement device according to claim 2,
wherein the measuring unit includes a distance-related-information measuring unit that measures, as the information relating to the target part, information that changes based on a distance to the target part from a specific position on optical paths of the laser beam and the signal light,
the optical member is present on an optical path shared by the laser beam and the signal light, and
based on the information measured by the distance-related-information measuring unit, the optical-member movable unit transfers the optical member along the shared optical path to a position where the region to which the laser beam is condensed is capable of matching the specific region in the subject portion in the target part, and where a focused image of the signal light from the region to which the laser beam is condensed can be formed on the photodetector,
whereafter the light emission unit emits the laser beam,
the photodetector receives the signal light based on the laser beam and detects the intensity of the signal light, and
the controller measures the concentration of the substance in blood based on the intensity of the signal light.

4. The substance-in-blood concentration measurement device according to claim 2,
wherein the measuring unit includes a distance measuring unit that measures, as the information relating to the target part, a distance to the target part from a specific position on optical paths of the laser beam and the signal light,
the optical member includes:
a first optical member that condenses the laser beam to the specific region in the subject portion in the target part; and
a second optical member that forms, on the photodetector, a focused image of the signal light from the region to which the laser beam is condensed in the subject portion,
the optical-member movable unit includes:
a first position changing unit that controls a position of the first optical member on the optical path of the laser beam; and
a second position changing unit that controls a position of the second optical member on the optical path of the signal light, and
based on the distance measured by the distance measuring unit, the first position changing unit transfers the first optical member to a position where the region to which the laser beam is condensed is capable of matching the specific region in the subject portion in the target part, and
based on the distance measured by the distance measuring unit, the second position changing unit transfers the second optical member to a position where a focused image of the signal light from the region to which the laser beam is condensed can be formed on the photodetector,
whereafter the light emission unit emits the laser beam,
the photodetector receives the signal light based on the laser beam and detects the intensity of the signal light, and
the controller measures the concentration of the substance in blood based on the intensity of the signal light.

5. The substance-in-blood concentration measurement device according to claim 2,
wherein the measuring unit includes a position measuring unit that measures, as the information relating to the target part, a position of the target part relative to a specific position on optical paths of the laser beam and the signal light,
the optical member includes:
a first optical member that is positioned on the optical path of the laser beam and that condenses the laser beam to the specific region in the subject portion in the target part; and
a second optical member that is positioned on the optical path of the signal light and that forms, on the photodetector, a focused image of the signal light from the region to which the laser beam is condensed in the subject portion in the target part,
the optical-member movable unit includes:
a first angle changing unit that changes an emission angle of the first optical member; and
a second angle changing unit that changes a light-receiving angle of the second optical member, and
based on the position measured by the position measuring unit, the first angle changing unit changes the emission angle of the first optical member to a direction in which the region to which the laser beam is condensed is capable of matching the specific region in the subject portion in the target part, and
based on the position measured by the position measuring unit, the second angle changing unit changes the light-receiving angle of the second optical member to a direction in which a focused image of the signal light from the region to which the laser beam is condensed can be formed on the photodetector,
whereafter the light emission unit emits the laser beam,
the photodetector receives the signal light based on the laser beam and detects the intensity of the signal light, and
the controller measures the concentration of the substance in blood based on the intensity of the signal light.

6. The substance-in-blood concentration measurement device according to claim 2,
wherein the measuring unit includes a detecting unit that detects, as the information relating to the target part, an area of presence of the target part of the subject person in a space,
the optical-member movable unit can change a position and an angle of the optical member with respect to each of a plurality of positions in the space adaptively so that the region to which the laser beam is condensed is capable of matching the specific region in the subject portion and a focused image of the signal light from the region to which the laser beam is condensed can be formed on the photodetector, and
based on the area of presence of the target part detected by the detecting unit, the optical-member movable unit selects a measurement-target position from among the plurality of positions and changes the position and the angle of the optical member so as to be adapted to the measurement-target position,
whereafter the light emission unit emits the laser beam,
the photodetector receives the signal light based on the laser beam and detects the intensity of the signal light, and
the controller measures the concentration of the substance in blood based on the intensity of the signal light.

7. The substance-in-blood concentration measurement device according to claim 1,
wherein the measuring unit includes a detecting unit that detects, as the information relating to the target part, an area of presence of the target part of the subject person in a space,
the optical-member movable unit can change a position and an angle of the optical member with respect to each of a plurality of positions in the space adaptively so that the region to which the laser beam is condensed is capable of matching the specific region in the subject portion in the target part and a focused image of the signal light from the region to which the laser beam is condensed can be formed on the photodetector, and
the detecting unit detects the area of presence of the target part of the subject person,
the optical-member movable unit changes the position and the angle of the optical member so as to be adapted to each of the plurality of positions,
to each of the plurality of positions, the light emission unit emits the laser beam,
from each of the plurality of positions, the photodetector receives signal light based on the laser beam and detects the intensity of the signal light, and
the controller measures the concentration of the substance in blood based on the intensity of the obtained signal light, and further selects a measurement-target position from among the plurality of positions based on the area of presence of the target part and selects the concentration of the substance in blood corresponding to the measurement-target position.

8. A substance-in-blood concentration measurement device that measures a concentration of a substance in blood included in the blood in a subject portion in a target part of a subject person, the substance-in-blood concentration measurement device comprising:
a detecting unit that detects whether or not the target part of the subject person is present within a specific area in a space;
a light emission unit that emits a laser beam to a specific region in the subject portion in the target part;
a photodetector that receives signal light from the subject person based on the laser beam and detects an intensity of the signal light;
an optical member that condenses the laser beam to the specific region in the subject portion in the target part located in the specific area, and forms, on the photodetector, a focused image of the signal light from a region to which the laser beam is condensed in the subject portion; and
a controller that, based on the intensity of the signal light, obtains a concentration of the substance in blood in the region to which the laser beam is condensed,
wherein, when the detecting unit detects that the target part is present within the specific area in the space,
the light emission unit emits the laser beam,
the photodetector receives the signal light based on the laser beam and detects the intensity of the signal light, and
the controller measures the concentration of the substance in blood based on the intensity of the signal light.

9. The substance-in-blood concentration measurement device according to any one of claims 1 to 8,
wherein positions of the light emission unit and the optical member relative to the subject portion are set so that a blood-vessel region located inward of the epidermis in the subject portion and the region to which the laser beam is condensed by the optical member overlap, and
positions of the photodetector and the optical member relative to the subject portion are set so that an image of the signal light from the blood-vessel region overlapping the region to which the laser beam is condensed is transferred by the optical member to form a focused image on a light receiving surface of the photodetector.

10. The substance-in-blood concentration measurement device according to any one of claims 1 to 8,
within a section from the light emission unit to the surface of the subject portion in an optical path from the light emission unit to the subject portion, the laser beam propagates through a space except within a section in which the laser beam passes through the optical member, and
within a section from the surface of the subject portion to a light receiving surface of the photodetector in an optical path from the subject portion to the photodetector, the signal light propagates through a space except within a section in which the signal light passes through the optical member.

11. A substance-in-blood concentration measurement method for measuring a concentration of a substance in blood included in the blood in a subject portion in a target part of a subject person, the substance-in-blood concentration measurement method comprising:
using a measuring unit and measuring information relating to the target part of the subject person;
based on the information relating to the target part, using an optical-member movable unit and changing a state of an optical member;
using a light emission unit and emitting a laser beam to a specific region in the subject portion in the target part;
using the optical member and condensing the laser beam to the specific region in the subject portion in the target part and forming, on a photodetector, a focused image of signal light from a region to which the laser beam is condensed in the subject portion;
using the photodetector and receiving the signal light from the subject person based on the laser beam and detecting an intensity of the signal light; and
based on the intensity of the signal light, obtaining a concentration of the substance in blood in the region to which the laser beam is condensed.

12. A program that causes a computer to execute substance-in-blood concentration measurement processing of measuring a concentration of a substance in blood included in the blood in a subject portion in a target part of a subject person,
wherein the substance-in-blood concentration measurement processing comprises:
using a measuring unit and measuring information relating to the target part of the subject person;
based on the information relating to the target part, using an optical-member movable unit and changing a state of an optical member;
using a light emission unit and emitting a laser beam to a specific region in the subject portion in the target part;
using the optical member and condensing the laser beam to the specific region in the subject portion in the target part and forming, on a photodetector, a focused image of signal light from a region to which the laser beam is condensed in the subject portion;
using the photodetector and receiving the signal light from the subject person based on the laser beam and detecting an intensity of the signal light; and
based on the intensity of the signal light, obtaining a concentration of the substance in blood in the region to which the laser beam is condensed.
